# EUROPEAN PATENT APPLICATION

(11) **EP 4 624 449 A1**
(43) Date of publication of application: **01.10.2025**
(21) Application number: 23893956.5
(22) Date of filing: 23.11.2023
(51) Int. Cl.: C07C 233/20, C07C 233/18, C07C 231/02, A61K 8/68, A61K 31/164, A61Q 19/00, A61Q 19/08, A61Q 19/02, A61P 17/02, A61P 17/00, A61P 39/06, A61P 29/00, A23L 33/12, A23L 33/105

(54) **VEGETABLE OIL CERAMIDES, SYNTHESIS METHOD THEREFOR, AND USE THEREOF**

(30) Priority: 25.11.2022 CN 202211493023; 02.02.2023 CN 202310051777; 02.02.2023 CN 202310051769; 02.02.2023 CN 202310051767; 02.02.2023 CN 202310051764; 02.02.2023 CN 202310051762; 02.02.2023 CN 202310051760; 02.02.2023 CN 202310051756; 02.02.2023 CN 202310051753; 02.02.2023 CN 202310051748; 02.02.2023 CN 202310051745; 02.02.2023 CN 202310051741; 02.02.2023 CN 202310051737; 02.02.2023 CN 202310051734; 02.02.2023 CN 202310051732; 02.02.2023 CN 202310051729; 02.02.2023 CN 202310051723; 02.02.2023 CN 202310051600; 02.02.2023 CN 202310051569; 02.02.2023 CN 202310051559; 02.02.2023 CN 202310051553; 02.02.2023 CN 202310051539; 02.02.2023 CN 202310051531; 02.02.2023 CN 202310051508; 02.02.2023 CN 202310051500; 02.02.2023 CN 202310051497; 02.02.2023 CN 202310051491; 01.03.2023 CN 202310186309; 01.03.2023 CN 202310186329; 01.03.2023 CN 202310186326; 01.03.2023 CN 202310186331; 01.03.2023 CN 202310186333; 01.03.2023 CN 202310186335; 01.03.2023 CN 202310186336
(71) Applicant: Shenzhen Dikeman Biotechnology Co., Ltd., Shenzhen, Guangdong 518000 (CN)
(72) Inventor: YANG, Chaowen, Shenzhen, Guangdong 518000 (CN); YE, Liu, Shenzhen, Guangdong 518000 (CN); LI, Qing, Shenzhen, Guangdong 518000 (CN)
(74) Representative: Global IP Europe Patentanwaltskanzlei
(86) International application number: PCT/CN2023/133524
(87) International publication number: WO 2024/109867

(57) **Abstract**

The invention belongs to the field of biomedicine technology, and discloses a plant oil ceramide, which is obtained by reacting a plant oil fatty acid with a sphingoid base compound, wherein the sphingoid base compound is selected from sphingosine, phytosphingosine, and dihydrosphingosine, and the plant oil fatty acid is selected from olive oil fatty acid, camellia oil fatty acid, sea buckthorn oil fatty acid, grape seed oil fatty acid, nut oil fatty acid, borage oil fatty acid, rosehip oil fatty acid, soybean oil fatty acid, rice bran oil fatty acid and the like. This invention also discloses a synthesis method and use of plant oil ceramide. Said plant oil ceramide exhibits outstanding performance in skin's natural barrier repair, anti-inflammatory activity, tissue healing, anti-aging effects, and more, with broad application prospects in cosmetics, health supplements, and biopharmaceuticals.

## Description

### Field of the Invention

The present invention belongs to the technical field of biological medicine, and specifically relates to plant oil ceramides, synthesis method and use thereof.

### Background of the Invention

Ceramides (also known as molecular nails) are naturally present in the skin and constitute a critical component of the skin barrier (stratum corneum), accounting for up to 40-50 wt% of its content. Ceramides are a type of sphingolipid composed of sphingoid long-chain bases and fatty acids, where variations in the sphingoid moiety, fatty acid chain length, degree of unsaturation, and number of hydroxyl groups result in a diverse class of compounds. Ceramides exhibit outstanding performance in regulating skin barrier function, restoring skin moisture, and enhancing adhesion between skin keratinocytes.

Given the importance of ceramides, many cosmetic and pharmaceutical companies are actively researching and developing related products. Natural plant-derived ceramides, owing to their sustainable and eco-friendly raw material sources, as well as their structural similarity to skin ceramides, may form an effective skin barrier to prevent moisture loss and combat external damage. These properties position them as potential next-generation environmentally friendly, safe, and reliable ceramide products.

Plant oils are extracted from oil-rich plant fruits, seeds, germ, or other parts through some pretreatment processes such as cleaning, impurity removal, dehulling, crushing, softening, flaking, extrusion, and solvent extraction or mechanical pressing to obtain crude oil, which is then refined. Fatty acids in plant oils moisturize and impart luster to the skin. The content and types of fatty acids vary across plant oils, which often also contain other physiologically active substances.

### Summary of the Invention

An object of the present invention is to provide a ceramide synthesized from plant oil fatty acids.

Another object of the present invention is to provide a method for synthesizing plant oil ceramides, utilizing natural plant oil and readily available plant oils or plant oil fatty acids as raw materials.

Another object of the present invention is to provide use of the plant oil ceramide.

In order to achieve one of the above objects, the present invention uses the following technical solutions.

In a first aspect of the present invention, plant oil ceramide is obtained by a reaction of a plant oil fatty acid with a sphingoid base compound, wherein the sphingoid base compound is selected from sphingosine, phytosphingosine, and dihydrosphingosine, and the plant oil fatty acid is selected from olive oil fatty acid, camellia oil fatty acid, sea buckthorn oil fatty acid, grape seed oil fatty acid, nut oil fatty acid, borage oil fatty acid, rosehip oil fatty acid, soybean oil fatty acid, rice bran oil fatty acid, cottonseed oil fatty acid, *Plukenetia volubilis* oil fatty acid, watermelon seed oil fatty acid, coconut oil fatty acid, *Acer truncatum* seed oil fatty acid, walnut oil fatty acid, avocado oil fatty acid, tomato seed oil fatty acid, *Ximenia americana* seed oil fatty acid, *Prinsepia utilis* oil fatty acid, safflower seed oil fatty acid, peony seed oil fatty acid, sunflower seed oil fatty acid, perilla seed oil fatty acid, linseed oil fatty acid, *Silybum marianum* oil fatty acid, shea butter fatty acid, evening primrose oil fatty acid, argan oil fatty acid, hemp seed oil fatty acid, meadowfoam seed oil fatty acid, baobab seed oil fatty acid, cactus seed oil fatty acid, wheat germ oil fatty acid, sesame oil fatty acid, corn oil fatty acid, almond oil fatty acid, *Malania oleifera* oil fatty acid, peanut oil fatty acid, blackcurrant seed oil fatty acid, rapeseed oil fatty acid, hazelnut oil fatty acid, pumpkin seed oil fatty acid, DHA algal oil fatty acid, crabapple oil fatty acid, kiwi seed oil fatty acid, marula oil fatty acid, European plum seed oil fatty acid.

The reaction can be a chemical synthesis reaction (as described in detail below), or a microbial fermentation method can be used, that is, using Pichia pastoris or Saccharomyces cerevisiae to ferment under certain conditions to obtain sphingoid base compounds, and then adding fatty acid to finally obtain ceramide; or using plant oil as raw material, selecting a suitable strain, and fermenting to obtain plant oil ceramide.

Sphingosine refers to 2-amino-4-octadecene-1,3-diol, phytosphingosine refers to 2-amino-octadecane-1,3,4-triol, and dihydrosphingosine refers to 2-amino-octadecane-1,3-diol.

Further, the plant oil fatty acid is obtained by hydrolysis of plant oil. For example, camellia oil fatty acid is obtained by hydrolysis of camellia oil, and sea buckthorn oil fatty acid is obtained by hydrolysis of sea buckthorn seed oil or sea buckthorn fruit oil.

Olive oil fatty acid is composed of 20-83 wt.% oleic acid, 7.5-20 wt.% palmitic acid, 3.5-70 wt.% linoleic acid, 0.5-5 wt.% stearic acid, 0.1-1.0 wt.% linolenic acid, 0-3.5 wt.% palmitoleic acid, 0-0.6 wt.% arachidic acid, 0-0.4 wt.% eicosenoic acid, 0-0.3 wt.% heptadecanoic acid, 0-0.3 wt.% heptadecenoic acid, 0-0.2 wt.% behenic acid, 0-0.2 wt.% lignoceric acid, 0-0.05 wt.% myristic acid.

Camellia oil fatty acid is composed of 75-90 wt.% oleic acid, 2-15 wt.% linoleic acid, 1-10 wt.% palmitic acid, 0.1-3 wt.% stearic acid, 0.01-1 wt.% linolenic acid, 0-0.5 wt.% arachidic acid.

Sea buckthorn oil fatty acid is composed of 20-35 wt.% oleic acid, 8-30 wt.% palmitic acid, 4-40 wt.% linoleic acid, 2-30 wt.% linolenic acid, 0.5-35 wt.% palmitoleic acid, 0.5-3 wt.% stearic acid, 0-1 wt.% myristic acid.

Grape seed oil fatty acid is composed of 55-85 wt.% linoleic acid, 8-25 wt.% oleic acid, 1-12 wt.% palmitic acid, 0.5-7 wt.% stearic acid, 0.01-2 wt.% palmitoleic acid, 0.01-1 wt.% linolenic acid, 0-1 wt.% arachidic acid.

Nut oil fatty acid is composed of 55-80 wt.% oleic acid, 12-35 wt.% palmitoleic acid, 2-10 wt.% palmitic acid, 0.4-3 wt.% linoleic acid, 0.1-3 wt.% arachidic acid, 0.2-5 wt.% stearic acid, 0.01-1 wt.% linolenic acid, 0.01-1 wt.% behenic acid, 0-1.5 wt.% myristic acid, 0-3 wt.% arachidonic acid.

Borage oil fatty acid is composed of 20-40 wt.% γ-linolenic acid, 30-45 wt.% linoleic acid, 15-25 wt.% oleic acid, 2-8 wt.% palmitic acid, 1-5 wt.% stearic acid, 0-2 wt.% arachidic acid, 0-2 wt.% arachidonic acid, 0-2 wt.% erucic acid.

Rosehip oil fatty acid is composed of 30-60 wt.% linoleic acid, 20-45 wt.% linolenic acid, 6-25 wt.% oleic acid, 0.1-3 wt.% stearic acid, 0.5-5 wt.% palmitic acid, 0.01-1 wt.% arachidic acid, 0-1 wt.% arachidonic acid.

Soybean oil fatty acid is composed of 50-75 wt.% linoleic acid, 15-30 wt.% oleic acid, 2-12 wt.% palmitic acid, 1-10 wt.% linolenic acid, 1-5 wt.% stearic acid, 0-2 wt.% arachidic acid, 0-2 wt.% behenic acid.

Rice bran oil fatty acid is composed of 40-60 wt.% oleic acid, 20-45 wt.% linoleic acid, 5-20 wt.% palmitic acid, 0.5-5 wt.% stearic acid, 0.1-2 wt.% linolenic acid, 0-2 wt.% arachidic acid.

Cottonseed oil fatty acid is composed of 55-80 wt.% linoleic acid, 10-25 wt.% palmitic acid, 6-18 wt.% oleic acid, 1-3 wt.% stearic acid, 0.1-1 wt.% arachidic acid, 0.02-1 wt.% palmitoleic acid, and 0.01-1 wt.% linolenic acid.

*Plukenetia volubilis* oil fatty acid is composed of 45-68 wt.% linolenic acid, 20-40 wt.% linoleic acid, 5-12 wt.% oleic acid, 2-5 wt.% palmitic acid, 1-4 wt.% stearic acid.

Watermelon seed oil fatty acid is composed of 60-80 wt.% linoleic acid, 3-15 wt.% palmitic acid, 10-30 wt.% oleic acid, 1-5 wt.% stearic acid.

Coconut oil fatty acid is composed of 20-70 wt.% lauric acid, 10-50 wt.% myristic acid, 10-20 wt.% palmitic acid, 2-10 wt.% stearic acid, 2-10 wt.% oleic acid, 0-3 wt.% decanoic acid, 0-3 wt.% caproic acid.

*Acer truncatum* seed oil fatty acid is composed of 25-48 wt.% linoleic acid, 20-40 wt.% oleic acid, 5-30 wt.% arachidic acid, 4-15 wt.% arachidonic acid, 0.5-5 wt.% linolenic acid, 0.1-5 wt.% palmitic acid, 0.1-4 wt.% stearic acid.

Walnut oil fatty acid is composed of 40-75 wt.% linoleic acid, 15-50 wt.% oleic acid, 2-10 wt.% palmitic acid, 0-5 wt.% linolenic acid, 0-4 wt.% stearic acid.

Avocado oil fatty acid is composed of 50-80 wt.% oleic acid, 5-25 wt.% palmitic acid, 5-20 wt.% linoleic acid, 6-18 wt.% palmitic acid, 0.1-2 wt.% stearic acid, 0.1-1 wt.% linolenic acid, 0-1 wt.% arachidic acid, 0-0.5 wt.% arachidonic acid.

Tomato seed oil fatty acid is composed of 55-80 wt.% linoleic acid, 10-25 wt.% oleic acid, 3-20 wt.% palmitic acid, 1-8 wt.% stearic acid, 0.2-2 wt.% linolenic acid, 0-1 wt.% arachidic acid.

*Ximenia americana* seed oil fatty acid is composed of 50-75 wt.% oleic acid, 15-40 wt.% linolenic acid, 0.5-10 wt.% stearic acid, 0-15 wt.% ximenynic acid, 0-15 wt.% nervonic acid, 0-2 wt.% behenic acid, 0-1 wt.% palmitic acid, 0-1 wt.% linoleic acid.

*Prinsepia utilis* oil fatty acid is composed of 30-50 wt.% linoleic acid, 25-40 wt.% oleic acid, 10-25 wt.% palmitic acid, 3-10 wt.% stearic acid, 0-1 wt.% palmitoleic acid, 0-1 wt.% arachidic acid.

Safflower seed oil fatty acid is composed of 70-90 wt.% linoleic acid, 4-20 wt.% oleic acid, 1-8 wt.% palmitic acid, 0.01-2 wt.% linolenic acid, 0-2 wt.% stearic acid, 0-1 wt.% arachidic acid.

Peony seed oil fatty acid is composed of 35-70 wt.% α-linolenic acid, 15-40 wt.% linoleic acid, 10-30 wt.% oleic acid, 0.1-5 wt.% palmitic acid, 0.1-5 wt.% stearic acid, 0-1 wt.% arachidic acid.

Sunflower seed oil fatty acid is composed of 55-75 wt.% linoleic acid, 20-40 wt.% oleic acid, 0.5-7 wt.% stearic acid, 1-8 wt.% palmitic acid, 0-2 wt.% behenic acid, 0-1 wt.% arachidic acid.

Perilla seed oil fatty acid is composed of 65-90 wt.% α-linolenic acid, 4-30 wt.% linoleic acid, 4-20 wt.% oleic acid, 1-4 wt.% stearic acid, 0-3.5 wt.% palmitic acid, 0-0.6 wt.% arachidonic acid.

Linseed oil fatty acid is composed of 40-70 wt.% linolenic acid, 10-25 wt.% linoleic acid, 10-25 wt.% oleic acid, 1-8 wt.% palmitic acid, 1-8 wt.% stearic acid, 0-1 wt.% arachidic acid.

*Silybum marianum* oil fatty acid is composed of 50-75 wt.% linoleic acid, 15-45 wt.% oleic acid, 1-10 wt.% palmitic acid, 1-6 wt.% stearic acid, 0.2-4 wt.% arachidic acid, 0.5-4 wt.% behenic acid, 0.01-2.5 wt.% linolenic acid, 0-1.5 wt.% myristic acid.

Shea butter fatty acid is composed of 35-60 wt.% oleic acid, 25-55 wt.% stearic acid, 2-10 wt.% linoleic acid, 0.5-10 wt.% palmitic acid, 0.1-2 wt.% arachidic acid, 0-1 wt.% linolenic acid, 0-1 wt.% behenic acid.

Evening primrose oil fatty acid is composed of 55-88 wt.% linoleic acid, 3-20 wt.% oleic acid, 5-20 wt.% γ-linolenic acid, 0.5-5 wt.% palmitic acid, 0-2 wt.% stearic acid, 0-2 wt.% arachidonic acid, 0-1 wt.% behenic acid.

Argan oil fatty acid is composed of 35-50 wt.% oleic acid, 30-50 wt.% linoleic acid, 5-15 wt.% palmitic acid, 2-8 wt.% stearic acid, 0.1-1 wt.% arachidic acid, 0.01-0.5 wt.% linolenic acid, 0-0.5 wt.% behenic acid, 0-0.5 wt.% arachidonic acid.

Hemp seed oil fatty acid is composed of 50-75 wt.% linoleic acid, 5-30 wt.% linolenic acid, 5-20 wt.% oleic acid, 3-15 wt.% palmitic acid, 1-5 wt.% stearic acid.

Meadowfoam seed oil fatty acid is composed of 50-80 wt.% cis-5-eicosenoic acid, 8-25 wt.% cis-5,13-docosadienoic acid, 5-20 wt.% erucic acid, 1-10 wt.% cis-5-docosenoic acid, 0-1 wt.% oleic acid, 0-1 wt.% linoleic acid.

Baobab seed oil fatty acid is composed of 30-50 wt.% oleic acid, 15-35 wt.% linoleic acid, 13-30 wt.% palmitic acid, 2-8 wt.% stearic acid, 0.5-5 wt.% linolenic acid, 0.2-2 wt.% arachidic acid, 0-1 wt.% palmitoleic acid, 0-0.5 wt.% myristic acid.

Cactus seed oil fatty acid is composed of 50-75 wt.% linolenic acid, 10-30 wt.% oleic acid, 5-15 wt.% palmitoleic acid, 3-10 wt.% palmitic acid, 3-8 wt.% stearic acid, 0.1-1 wt.% arachidic acid.

Wheat germ oil fatty acid is composed of 50-75 wt.% linoleic acid, 15-45 wt.% oleic acid, 2-10 wt.% palmitic acid, 1-8 wt.% linolenic acid, 0.1-1 wt.% arachidonic acid, 0-0.5 wt.% stearic acid, 0-0.5 wt.% arachidic acid.

Sesame oil fatty acid is composed of 45-70 wt.% linoleic acid, 15-35 wt.% oleic acid, 6-20 wt.% palmitic acid, 0-2 wt.% stearic acid, 0-1 wt.% arachidic acid, 0-0.5 wt.% linolenic acid.

Corn oil fatty acid is composed of 50-70 wt.% linoleic acid, 20-35 wt.% oleic acid, 5-18 wt.% palmitic acid, 0.5-3 wt.% stearic acid, 0.01-1 wt.% linolenic acid, 0-1 wt.% arachidic acid.

Almond oil fatty acid is composed of 55-80 wt.% oleic acid, 15-35 wt.% linoleic acid, 0.1-8 wt.% palmitic acid, 0.5-2 wt.% stearic acid, 0.01-1 wt.% linolenic acid.

Garlic fruit oil fatty acid is composed of 40-60 wt.% nervonic acid, 30-50 wt.% oleic acid, 5-12 wt.% arachidic acid, 0-2 wt.% palmitic acid, 0-2 wt.% linoleic acid.

Peanut oil fatty acid is composed of 40-60 wt.% linoleic acid, 30-50 wt.% oleic acid, 4-12 wt.% palmitic acid, 0.5-5 wt.% stearic acid, 0-1 wt.% arachidic acid, 0-1 wt.% behenic acid.

Blackcurrant seed oil fatty acid is composed of 40-60 wt.% linoleic acid, 20-40 wt.% linolenic acid, 10-20 wt.% oleic acid, 1-8 wt.% palmitic acid, 0-2 wt.% stearic acid, 0-2 wt.% arachidic acid.

Rapeseed oil fatty acid is composed of 50-80 wt.% oleic acid, 10-30 wt.% linoleic acid, 2-8 wt.% linolenic acid, 1-5 wt.% stearic acid, 0.5-5 wt.% palmitic acid, 0-1 wt.% arachidic acid, 0-1 wt.% arachidonic acid, 0-1 wt.% erucic acid.

Hazelnut oil fatty acid is composed of 70-90 wt.% oleic acid, 8-25 wt.% linoleic acid, 0.5-5 wt.% palmitic acid, 0.5-4 wt.% stearic acid, 0-0.5 wt.% linolenic acid, 0-0.5 wt.% palmitoleic acid.

Pumpkin seed oil fatty acid is composed of 40-70 wt.% linoleic acid, 20-55 wt.% oleic acid, 3-10 wt.% palmitic acid, 1-5 wt.% stearic acid, 0.1-5 wt.% linolenic acid, 0-1 wt.% arachidic acid, 0-0.5 wt.% palmitoleic acid.

DHA algal oil fatty acid is composed of 50-75 wt.% DHA, 15-35 wt.% oleic acid, 3-15 wt.% palmitic acid, 0.1-1 wt.% stearic acid, 0.1-1 wt.% EPA, 0.01-0.5 wt.% linolenic acid, 0-0.5 wt.% arachidic acid, 0-0.5 wt.% behenic acid.

Crabapple oil fatty acid is composed of 30-50 wt.% oleic acid, 15-40 wt.% linoleic acid, 8-20 wt.% stearic acid, 6-20 wt.% palmitic acid, 0.2-1.5 wt.% arachidic acid, 0.05-2 wt.% linolenic acid, 0.05-1 wt.% palmitoleic acid, 0-1 wt.% behenic acid, 0-0.5 wt.% arachidonic acid.

Kiwi seed oil fatty acid is composed of 45-70 wt.% linolenic acid, 8-20 wt.% linoleic acid, 5-20 wt.% oleic acid, 2-10 wt.% palmitic acid, 0.5-5 wt.% stearic acid.

Marula oil fatty acid is composed of 65-85 wt.% oleic acid, 5-15 wt.% palmitic acid, 2-10 wt.% linoleic acid, 2-10 wt.% stearic acid, 0.2-2 wt.% arachidic acid, 0-1 wt.% arachidonic acid, 0-0.5 wt.% linolenic acid.

European plum seed oil fatty acid is composed of 60-80 wt.% oleic acid, 10-30 wt.% linoleic acid, 0.5-5 wt.% stearic acid, 0.5-3 wt.% palmitoleic acid, 0-7 wt.% palmitic acid, 0-1 wt.% linolenic acid.

Plant oil fatty acids are affected by plant variety, soil, climate, origin, harvesting season, and extraction process, and the content of each component may vary. For example, the main component of olive oil fatty acid is oleic acid, and other fatty acids include linoleic acid, palmitic acid, stearic acid, and linolenic acid, which are necessary components, while palmitoleic acid, arachidic acid, eicosenoic acid, heptadecanoic acid, heptadecenoic acid, behenic acid, lignoceric acid, and myristic acid may not be contained and are optional or non-essential components.

The plant oil ceramide is selected from at least two of oleic acid ceramide, linoleic acid ceramide, palmitic acid ceramide, linolenic acid ceramide, stearic acid ceramide, palmitoleic acid ceramide, arachidic acid ceramide, arachidonic acid ceramide, behenic acid ceramide, erucic acid ceramide, myristic acid ceramide, nervonic acid, lauric acid ceramide, eicosenoic acid ceramide, heptadecanoic acid ceramide, heptadecenoic acid ceramide, lignoceric acid ceramide, DHA ceramide, EPA ceramide, decanoic acid ceramide, caproic acid ceramide, ximenynic acid ceramide, cis-5-eicosenoic acid ceramide, cis-5,13-docosadienoic acid ceramide, and cis-5-docosenoic acid ceramide.

Specifically, the plant oil ceramide is selected from at least two of no more than 90 wt.% oleic acid ceramide, no more than 90 wt.% linoleic acid ceramide, no more than 30 wt.% palmitic acid ceramide, no more than 90 wt.% linolenic acid ceramide, no more than 55 wt.% stearic acid ceramide, no more than 35 wt.% palmitoleic acid ceramide, no more than 30 wt.% arachidic acid ceramide, no more than 15 wt.% arachidonic acid ceramide, no more than 4 wt.% behenic acid ceramide, no more than 20 wt.% erucic acid ceramide, no more than 50 wt.% myristic acid ceramide, no more than 60 wt.% nervonic acid ceramide, no more than 70 wt.% lauric acid ceramide, no more than 0.4 wt.% eicosenoic acid ceramide, no more than 0.3 wt.% heptadecanoic acid ceramide, no more than 0.3 wt.% heptadecenoic acid ceramide, no more than 0.2 wt.% lignoceric acid ceramide, no more than 75 wt.% DHA ceramide, no more than 1 wt.% EPA ceramide, no more than 3 wt.% decanoic acid ceramide, no more than 3 wt.% caproic acid ceramide, no more than 15 wt.% ximenynic acid ceramide, no more than 80 wt.% cis-5-eicosenoic acid ceramide, no more than 25 wt.% cis-5,13-docosadienoic acid ceramide, and no more than 10 wt.% cis-5-docosenoic acid ceramide.

Linolenic acid ceramide refers to γ-linolenic acid ceramide and/or α-linolenic acid ceramide.

The plant oil ceramide also contains one or more compounds present in the plant oil fatty acids but not reacting with sphingoid base compounds, such as vitamin A, vitamin B, vitamin C, vitamin D, vitamin E, vitamin K, proanthocyanidins, carotenoids (β-carotene), triacontanol, phytosterols, squalene, tea polyphenols, camellia glycosides, paeonol, saponins, polysaccharides, flavonoids, caffeic acid, vanillic acid, tyrosol, resorcinol, resveratrol, fruit acid, tocopherol, allantoin, lecithin (phospholipids), alkaloids, thymol, sterols (β-sitosterol, campesterol, phytosterol), amylase, maltase, protease, lectin, oryzanol, triterpenoid esters, triterpenes, aromatic acids, lycopene, bipyranocoumarins, amino acids, trace elements.

Oleic acid ceramide is obtained by condensation reaction of oleic acid and sphingoid base compound, including oleic acid phytosphingosine ceramide, oleic acid sphingosine ceramide, and oleic acid dihydrosphingosine ceramide. Linoleic acid ceramide is obtained by condensation reaction of linoleic acid and sphingoid base compound, including linoleic acid phytosphingosine ceramide, linoleic acid sphingosine ceramide, and linoleic acid dihydrosphingosine ceramide. Palmitic acid ceramide is obtained by condensation reaction of palmitic acid and sphingoid base compounds, including palmitic acid phytosphingosine ceramide, palmitic acid sphingosine ceramide, and palmitic acid dihydrosphingosine ceramide. Other ceramides are deduced by analogy.

In a second aspect of the present invention, a method for synthesizing plant oil ceramide, comprising the following steps:
Under the condition of a condensing agent and an organic base, reacting a plant oil fatty acid with a sphingoid base compound, wherein the condensing agent is DCC or EDCI, and the organic base is DMAP, DIPEA, NMM or Et₃N.

Further, the molar ratio of the plant oil fatty acid, sphingoid base compound, condensing agent, and organic base is 1 : (1-1.5) : (1-2) : (0.2-2), and the reaction solvent is at least one of dichloromethane, tetrahydrofuran, ethyl acetate, and acetonitrile.

The plant oil purchased on the market is generally in the form of oil and fat, and needs to be hydrolyzed into plant oil fatty acids, so the following steps are also included:
The plant oil fat is hydrolyzed by saponification reaction to obtain plant oil fatty acids.

Further, the saponification reaction is the hydrolysis of the plant oil fat in potassium hydroxide solution.

Further, a mass ratio of the plant oil fat to potassium hydroxide is 1: (1-2).

A third aspect of the present invention relates to use of plant oil ceramides in cosmetics, medicines, dietary products or health products.

Further, the plant oil ceramides exhibit at least one of the following effects: skin barrier repair, tissue healing, anti-aging, anti-inflammatory, anti-photoaging, antioxidant activity, promotion of collagen synthesis, maintenance of elastin activity, and skin whitening.

A composition comprising plant oil ceramides, and the composition exhibiting at least one of the following effects: skin barrier repair, tissue healing, anti-aging, anti-inflammatory, anti-photoaging, antioxidant activity, promotion of collagen synthesis, maintenance of elastin activity, and skin whitening.

The composition contains acceptable excipients, including one or more of solubilizers, preservatives, antioxidants, pH adjusters, penetration enhancers, liposomes, humectants, thickeners, chelating agents, skin feel modifiers, surfactants, emulsifiers, fragrances and colorants. The composition is formulated as a cream, emulsion, solution, film, aerosol, or spray.

The present invention involves the following beneficial effects:
The fatty acids of plant oil and sphingoid base compounds naturally present in the skin are mildly reacted to prepare plant oil ceramides, which exhibit excellent performance in skin barrier repair, antioxidant activity, anti-aging and more, with broad application prospects in the fields of cosmetics, health products, biomedicines and other fileds.
1. Superior efficacy compared to single-component ceramides. Different ceramides exhibit varying effects due to structural differences, and single-structure ceramides generally lack comprehensive functionality. This invention adopts a biomimetic approach, utilizing natural plant oils or fatty acids to synthesize composite ceramides that compensate for efficacy gaps. Trace fatty acids in plant oils can form trace ceramides, providing supplemental benefits.
2. Enhanced performance compared to blended ceramides. Beyond fatty acids (or oils), plant oils contain vitamins A, carotenoids, resveratrol, tocopherols, sterols, amino acids, trace elements, and other physiologically active nutrients. The ceramides synthesized from plant oils synergize with these components, delivering superior effects compared to mechanically blended ceramides at similar ratios.
3. Lower cost. The method of the present invention can quickly obtain a composition compounded by multiple ceramides. Plant oils or fatty acids thereof are widely available, commercially accessible, and cost-effective, offering an eco-friendly and economical alternative to the idea of blending individually produced single-component ceramides. The single-component fatty acid not only has a high price, but also requires the production of different ceramides separately and then compounding, which increases the preparation cost.
4. Simplified synthesis. The method enables one-step preparation of multiple ceramides via chemical synthesis or microbial fermentation.

### Brief Description of the Drawings

Figures 1 and 2 are bar graphs of the test results of the cell proliferation activity of Example 37;
Figures 3, 4, and 5 are the test results of the cell migration ability of Example 38;
Figures 6, 7, and 8 are bar graphs of the elastase inhibition rate of Example 39;
Figures 9, 10, and 11 are bar graphs of the IL-6 factor expression level of the anti-inflammatory and repair efficacy test of Example 40;
Figures 12, 13, 14, 15, 16, and 17 are bar graphs of the MMP1 expression level of the anti-photoaging test of Example 41;
Figures 18, 19, 20, and 21 are bar graphs of the DPPH free radical scavenging rate of the antioxidant test of Example 42;
Figures 22, 23, and 24 are bar graphs of the melanin content of the whitening activity test of Example 43.

### Detailed Description of the Invention

The present invention is further described below in conjunction with specific examples. DCC refers to N,N'-dicyclohexylcarbodiimide, EDCI refers to 1-ethyl-(3-dimethylaminopropyl)carbodiimide, DMAP refers to 4-dimethylaminopyridine, Et₃N refers to triethylamine, DIPEA refers to N,N-diisopropylethylamine, and NMM refers to N-methylmorpholine. Silica gel column chromatography uses Qingdao Ocean Silica Gel (particle size 0.040-0.063 mm). Thin layer chromatography (TLC) uses 60F254 silica gel plate, and TLC color development uses UV light (254 nm) or iodine.

### Example 1

### Synthesis of ceramide from olive oil fatty acid and sphingosine

Step 1: 50 g of olive oil fat was dissolved in 60 mL of tetrahydrofuran, cooled in an ice bath, and then 100 mL of potassium hydroxide (25 wt.%) solution was added dropwise. After the addition was complete, it was heated to room temperature for reaction, until the reaction was complete by TLC detection.

Post-treatment: Dilute hydrochloric acid (3N) was added to adjust the pH value of the reaction system to 3. 150 mL of ethyl acetate was added to extract the aqueous phase. 100 mL of saturated brine was added to wash once, and then anhydrous Na₂SO₄ was added to dry the organic phase, then filtered and concentrated in vacuum to obtain 40g of olive oil fatty acid.

Step 2: 250 mL of olive oil fatty acid (50 mmol, molecular weight based on oleic acid), EDCI (75 mmol), and Et₃N (75 mmol) were added into a 250 mL round-bottom flask, then 100 mL of dichloromethane was added, followed by stirring at room temperature for 1 hour. Then, sphingosine (50 mmol) was added to the reaction system, followed by stirring at room temperature, until the reaction was complete by TLC detection.

Post-treatment: Water was added to quench the reaction. The organic layer was separated, dried, filtered, concentrated in vacuum, and then washed with solvent to obtain olive oil ceramide. The product was analyzed by HPLC. HPLC chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 2 wt.% water + 98 wt.% methanol, both contain 0.1 wt.% formic acid

The HPLC retention times of the main components were 20.7 min for oleic acid-sphingosine ceramide, 16.6 min for linoleic acid-sphingosine ceramide, respectively.

The resulting product was analyzed by high performance liquid chromatography. The mass ratios of oleic acid-sphingosine ceramide, palmitic acid-sphingosine ceramide, linoleic acid-sphingosine ceramide, stearic acid-sphingosine ceramide, and linolenic acid-sphingosine ceramide (H NMR spectrum was consistent with the standard spectrum) were 61.8%, 12.6%, 19.3%, 3.4%, and 0.6%, respectively, and the rest was other components with trace amounts. Imported olive oil fatty acid, the main component of which is oleic acid, was used in this example, and The resulting product was mainly oleic acid ceramide.

Oleic acid-sphingosine ceramide: ¹H NMR (400 MHz, Methanol-d4) δ 7.70 (d, J = 8.9 Hz, 1H), 5.69 (dt, J = 15.3, 6.7 Hz, 1H), 5.45 (dd, J = 15.4, 7.5 Hz, 1H), 5.34 (t, J = 4.9 Hz, 2H), 4.03 (t, J = 7.5 Hz, 1H), 3.85 (ddd, J = 9.5, 4.6, 2.4 Hz, 1H), 3.68 (d, J = 5.0 Hz, 2H), 2.19 (t, J = 7.6 Hz, 2H), 2.03 (q, J = 6.9, 6.0 Hz, 2H), 1.64-1.51 (m, 2H), 1.30 (d, J = 14.5 Hz, 41H), 0.90 (t, J = 6.7 Hz, 6H). Linoleic acid-sphingosine ceramide: ¹H NMR (400 MHz, Methanol-d4) δ 7.69 (d, J = 8.9 Hz, 1H), 5.69 (dt, J = 15.3, 6.7 Hz, 1H), 5.46 (dd, J = 15.3, 7.4 Hz, 1H), 5.41-5.25 (m, 4H), 4.03 (t, J = 7.5 Hz, 1H), 3.85 (tdd, J = 7.5, 4.7, 2.2 Hz, 1H), 3.68 (d, J = 5.0 Hz, 2H), 2.77 (t, J = 6.4 Hz, 2H), 2.20 (q, J = 7.6, 6.9 Hz, 2H), 2.13-1.94 (m, 6H), 1.67-1.49 (m, 2H), 1.31 (d, J = 19.6 Hz, 40H), 0.90 (td, J = 6.8, 3.8 Hz, 6H).

The synthesis of ceramide from olive oil fatty acid with phytosphingosine or dihydrosphingosine was carried out with reference to this example.

### Example 2

### Synthesis of ceramide from camellia oil fatty acid and phytosphingosine

Step 1: 50 g of camellia oil was dissolved in 60 mL of tetrahydrofuran, cooled in an ice bath, and then 100 mL of potassium hydroxide (25 wt.%) solution was added dropwise. After the addition was complete, it was heated to room temperature for reaction, until the reaction was complete by TLC detection.

Post-treatment: Dilute hydrochloric acid (3N) was added to adjust the pH value of the reaction system to 3. 150 mL of ethyl acetate was added to extract the aqueous phase. 100 mL of saturated brine was added to wash once, and then anhydrous Na₂SO₄ was added to dry the organic phase, then filtered and concentrated in vacuum to obtain 40.3 g of camellia oil fatty acid.

Step 2: Camellia oil fatty acid (50 mmol, based on the main fatty acid), DCC (60 mmol), and DMAP (10 mmol) were added into a 250 mL round-bottom flask, then 100 mL of dichloromethane was added, followed by stirring at room temperature for 1 hour. Then, phytosphingosine (60 mmol) was added to the reaction system, followed by stirring at room temperature, until the reaction was complete by TLC detection.

Post-treatment: The reaction liquid was filtered to remove solids, and the filtrate was collected and washed with saturated sodium hydrogen carbonate solution, 1N dilute hydrochloric acid and saturated brine, respectively. The organic layer was separated, dried, filtered and concentrated in vacuum, and then washed with solvent to obtain camellia oil ceramide. The product was analyzed by HPLC. HPLC chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for linolenic acid-phytosphingosine ceramide, 9.5 min for linoleic acid-phytosphingosine ceramide, 10.7 min for palmitic acid-phytosphingosine ceramide, 11.3 min for oleic acid-phytosphingosine ceramide, 13.9 min for stearic acid-phytosphingosine ceramide, respectively.

The resulting product was analyzed by high performance liquid chromatography. The contents of oleic acid-phytosphingosine ceramide, linoleic acid-phytosphingosine ceramide, palmitic acid-phytosphingosine ceramide, stearic acid-phytosphingosine ceramide, and linolenic acid-phytosphingosine ceramide were 77%, 13%, 3%, 2%, and 1%, respectively, and the rest was other components with trace amounts.

The synthesis of ceramide from camellia oil fatty acid with sphingosine or dihydrosphingosine was carried out with reference to this example.

### Example 3

### Synthesis of ceramide from sea buckthorn oil fatty acid and dihydrosphingosine

Step 1: 50 g of sea buckthorn seed oil was dissolved in 60 mL of tetrahydrofuran, cooled in an ice bath, and then 100 mL of potassium hydroxide (25 wt.%) solution was added dropwise. After the addition was complete, it was heated to room temperature for reaction, until the reaction was complete by TLC detection.

Post-treatment: Dilute hydrochloric acid (3N) was added to adjust the pH value of the reaction system to 3. 150 mL of ethyl acetate was added to extract the aqueous phase. 100 mL of saturated brine was added to wash once, and then anhydrous Na₂SO₄ was added to dry the organic phase, then filtered and concentrated in vacuum to obtain 39.6 g of sea buckthorn oil fatty acid.

Step 2: Sea buckthorn oil fatty acid (50 mmol, based on the main fatty acid), EDCI (80 mmol), and Et₃N (80 mmol) were added into a 250 mL round-bottom flask, then 100 mL of dichloromethane was added, followed by stirring at room temperature for 1 hour. Then dihydrosphingosine (60 mmol) was added to the reaction system, followed by stirring at room temperature, until the reaction was complete by TLC detection.

Post-treatment: Water was added to quench the reaction. The organic layer was separated, dried, filtered, concentrated in vacuum, and then washed with solvent to obtain sea buckthorn oil ceramide. The product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for linolenic acid-dihydrosphingosine ceramide, 9.0 min for palmitoleic acid-dihydrosphingosine ceramide, 9.6 min for linoleic acid-dihydrosphingosine ceramide, 10.6 min for palmitic acid-dihydrosphingosine ceramide, 11.1 min for oleic acid-dihydrosphingosine ceramide, 13.6 min for stearic acid-dihydrosphingosine ceramide, respectively.

The resulting product was analyzed by high performance liquid chromatography. The contents of oleic acid-dihydrosphingosine ceramide, palmitic acid-dihydrosphingosine ceramide, linoleic acid-dihydrosphingosine ceramide, linolenic acid-dihydrosphingosine ceramide, palmitoleic acid -dihydrosphingosine ceramide, and stearic acid-dihydrosphingosine ceramide were 28%, 27%, 6%, 25%, 7%, and 1%, respectively, and the rest was other components with trace amounts.

The synthesis of ceramide from sea buckthorn oil fatty acid with sphingosine or phytosphingosine was carried out with reference to this example.

### Example 4

### Synthesis of ceramide from grape seed oil fatty acid and phytosphingosine

Step 1: 50 g of grape seed oil was dissolved in 60 mL of tetrahydrofuran, cooled in an ice bath, and then 100 mL of potassium hydroxide (25 wt.%) solution was added dropwise. After the addition was complete, it was heated to room temperature for reaction, until the reaction was complete by TLC detection.

Post-treatment: Dilute hydrochloric acid (3N) was added to adjust the pH value of the reaction system to 3. 150 mL of ethyl acetate was added to extract the aqueous phase. 100 mL of saturated brine was added to wash once, and then anhydrous Na₂SO₄ was added to dry the organic phase, then filtered and concentrated in vacuum to obtain 40 g of grape seed oil fatty acid.

Step 2: Grape seed oil fatty acid (50 mmol, based on the main fatty acid), EDCI (60 mmol), and DIPEA (60 mmol) were added into a 250 mL round-bottom flask, then 100 mL of dichloromethane was added, followed by stirring at room temperature for 1 hour. Then phytosphingosine (60 mmol) was added to the reaction system, followed by stirring at room temperature, until the reaction was complete by TLC detection.

Post-treatment: Water was added to quench the reaction. The organic layer was separated, dried, filtered, concentrated in vacuum, and then washed with solvent to obtain grape seed oil ceramide. The product was analyzed by HPLC. HPLC chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.0 min for palmitoleic acid-phytosphingosine ceramide, 8.3 min for linolenic acid-phytosphingosine ceramide, 9.4 min for linoleic acid-phytosphingosine ceramide, 10.6 min for palmitic acid-phytosphingosine ceramide, 11.2 min for oleic acid-phytosphingosine ceramide, 13.8 min for stearic acid-phytosphingosine ceramide, respectively.

The resulting product was analyzed by high performance liquid chromatography. The contents of linoleic acid-phytosphingosine ceramide, oleic acid-phytosphingosine ceramide, palmitic acid-phytosphingosine ceramide, stearic acid-phytosphingosine ceramide, palmitoleic acid-phytosphingosine ceramide, and linolenic acid-phytosphingosine ceramide were 61%, 22%, 7%, 3%, 1%, and 1%, respectively, and the rest was other components with trace amounts.

The synthesis of ceramide from grape seed oil fatty acid with sphingosine or dihydrosphingosine was carried out with reference to this example.

### Example 5

With reference to Example 2, nut oil fatty acid and sphingosine were reacted to synthesize nut oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 7.9 min for linolenic acid-sphingosine ceramide, 8.2 min for palmitoleic acid-sphingosine ceramide, 8.7 min for linoleic acid-sphingosine ceramide, 10.2 min for oleic acid-sphingosine ceramide, 10.4 min for palmitic acid-sphingosine ceramide, 12.8 min for arachidonic acid-sphingosine ceramide, 13.6 min for stearic acid-sphingosine ceramide, 17.8 min for arachidic acid-sphingosine ceramide, 24.0 min for behenic acid-sphingosine ceramide, respectively.

The resulting product was analyzed by high performance liquid chromatography. The contents of oleic acid-sphingosine ceramide, palmitoleic acid-sphingosine ceramide, palmitic acid-sphingosine ceramide, linoleic acid-sphingosine ceramide, arachidic acid-sphingosine ceramide, stearic acid-sphingosine ceramide, linolenic acid-sphingosine ceramide, behenic acid-sphingosine ceramide, and arachidonic acid-sphingosine ceramide were 58%, 24%, 5%, 3%, 1%, 4%, 0.5%, 0.5%, and 2%, respectively, and the rest was other components with trace amounts.

The synthesis of ceramide from nut oil fatty acid with phytosphingosine or dihydrosphingosine was carried out with reference to this example.

### Example 6

With reference to Example 3, borage oil fatty acid and dihydrosphingosine were reacted to synthesize borage oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for γ-linolenic acid-dihydrosphingosine ceramide, 9.5 min for linoleic acid-dihydrosphingosine ceramide, 10.6 min for palmitic acid-dihydrosphingosine ceramide, 11.2 min for oleic acid-dihydrosphingosine ceramide, 13.5 min for stearic acid-dihydrosphingosine ceramide, 18.7 min for erucic acid-dihydrosphingosine ceramide, respectively.

The resulting product was analyzed by high performance liquid chromatography. The contents of γ-linolenic acid-dihydrosphingosine ceramide, linoleic acid-dihydrosphingosine ceramide, oleic acid-dihydrosphingosine ceramide, palmitic acid-dihydrosphingosine ceramide, stearic acid-dihydrosphingosine ceramide, and erucic acid-dihydrosphingosine ceramide were 27%, 44%, 19%, 4%, 2%, and 2%, respectively, and the rest was other components with trace amounts.

The synthesis of ceramide from borage oil fatty acid with sphingosine or phytosphingosine was carried out with reference to this example.

### Example 7

With reference to Example 2, rosehip oil fatty acid and phytosphingosine were reacted to synthesize rosehip oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for linolenic acid-phytosphingosine ceramide, 9.5 min for linoleic acid-phytosphingosine ceramide, 10.6 min for palmitic acid-phytosphingosine ceramide, 11.2 min for oleic acid-phytosphingosine ceramide, 12.9 min for arachidonic acid-phytosphingosine ceramide, 13.9 min for stearic acid-phytosphingosine ceramide, 16.5 min for arachidic acid-phytosphingosine ceramide, respectively.

The resulting product was analyzed by high performance liquid chromatography. The contents of linoleic acid-phytosphingosine ceramide, linolenic acid-phytosphingosine ceramide, oleic acid-phytosphingosine ceramide, stearic acid-phytosphingosine ceramide, palmitic acid-phytosphingosine ceramide, arachidic acid-phytosphingosine ceramide, and arachidonic acid-phytosphingosine ceramide were 51%, 26%, 10%, 3%, 4%, 0.5%, and 1%, respectively, and the rest was other components with trace amounts.

The synthesis of ceramide from rosehip oil fatty acid with sphingosine or dihydrosphingosine was carried out with reference to this example.

### Example 8

### Synthesis of ceramide from soybean oil fatty acid and sphingosine

Soybean oil fatty acid (50 mmol, based on the main fatty acid), EDCI (65 mmol), and DIPEA (65 mmol) were added into a 250 mL round-bottom flask, and then 100 mL of dichloromethane was added, followed by stirring at room temperature for 1 hour. Then sphingosine (60 mmol) was added to the reaction system, followed by stirring at room temperature, until the reaction was complete by TLC detection.

Post-treatment: Water was added to quench the reaction. The organic layer was separated, dried, filtered, concentrated in vacuum, and then washed with solvent to obtain soybean oil ceramide. The product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 7.9 min for linolenic acid-sphingosine ceramide, 8.7 min for linoleic acid-sphingosine ceramide, 10.2 min for oleic acid-sphingosine ceramide, 10.5 min for palmitic acid-sphingosine ceramide, 13.5 min for stearic acid-sphingosine ceramide, respectively.

The resulting product was analyzed by high performance liquid chromatography. The contents of linoleic acid-sphingosine ceramide, oleic acid-sphingosine ceramide, palmitic acid-sphingosine ceramide, linolenic acid-sphingosine ceramide, and stearic acid-sphingosine ceramide were 52%, 26%, 11%, 7%, and 2%, respectively, and the rest was other components with trace amounts.

The synthesis of ceramide from soybean oil fatty acid with phytosphingosine or dihydrosphingosine was carried out with reference to this example.

### Example 9

With reference to Example 8, rice bran oil fatty acid and dihydrosphingosine were reacted to synthesize rice bran oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for linolenic acid-dihydrosphingosine ceramide, 9.5 min for linoleic acid-dihydrosphingosine ceramide, 10.5 min for palmitic acid-dihydrosphingosine ceramide, 11.1 min for oleic acid-dihydrosphingosine ceramide, 13.5 min for stearic acid-dihydrosphingosine ceramide, and 16.1 min for arachidic acid-dihydrosphingosine ceramide, respectively.

The resulting product was analyzed by high performance liquid chromatography. The contents of oleic acid-dihydrosphingosine ceramide, linoleic acid-dihydrosphingosine ceramide, palmitic acid-dihydrosphingosine ceramide, stearic acid-dihydrosphingosine ceramide, linolenic acid-dihydrosphingosine ceramide, and arachidic acid-dihydrosphingosine ceramide were 54%, 21%, 17%, 3%, 1%, and 2%, respectively, and the rest was other components with trace amounts.

The synthesis of ceramide from rice bran oil fatty acid with sphingosine or phytosphingosine was carried out with reference to this example.

### Example 10

### Synthesis of ceramide from cottonseed oil fatty acid and phytosphingosine

Step 1: 50 g of cottonseed oil was dissolved in 60 mL of tetrahydrofuran, cooled in an ice bath, and then 100 mL of potassium hydroxide (25 wt.%) solution was added dropwise. After the addition was complete, it was heated to room temperature for reaction, until the reaction was complete by TLC detection.

Post-treatment: Dilute hydrochloric acid (3N) was added to adjust the pH value of the reaction system to 3. 150 mL of ethyl acetate was added to extract the aqueous phase. 100 mL of saturated brine was added to wash once, and then anhydrous Na₂SO₄ was added to dry the organic phase, then filtered and concentrated in vacuum to obtain 40.5 g of cottonseed oil fatty acid.

Step 2: Cottonseed oil fatty acid (50 mmol, based on the main fatty acid), EDCI (65 mmol), and NMM (65 mmol) were added into a 250 mL round-bottom flask, then 100 mL of dichloromethane was added, followed by stirring at room temperature for 1 hour. Then phytosphingosine (55 mmol) was added to the reaction system, followed by stirring at room temperature, until the reaction was complete by TLC detection.

Post-treatment: Water was added to quench the reaction. The organic layer was separated, dried, filtered, concentrated in vacuum, and then washed with solvent to obtain cottonseed oil ceramide. The product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.1 min for palmitoleic acid-phytosphingosine ceramide, 8.3 min for linolenic acid-phytosphingosine ceramide, 9.5 min for linoleic acid-phytosphingosine ceramide, 10.7 min for palmitic acid-phytosphingosine ceramide, 11.3 min for oleic acid-phytosphingosine ceramide, 13.9 min for stearic acid-phytosphingosine ceramide, 16.5 min for arachidic acid-phytosphingosine ceramide, respectively.

The resulting product was analyzed by high performance liquid chromatography. The contents of linoleic acid-phytosphingosine ceramide, palmitic acid-phytosphingosine ceramide, oleic acid-phytosphingosine ceramide, stearic acid-phytosphingosine ceramide, arachidic acid-phytosphingosine ceramide, palmitoleic acid-phytosphingosine ceramide, and linolenic acid-phytosphingosine ceramide were 74%, 12%, 7%, 2%, 1%, 1%, and 1%, respectively, and the rest was other components with trace amounts.

The synthesis of ceramide from cottonseed oil fatty acid with sphingosine or dihydrosphingosine was carried out with reference to this example.

### Example 11

With reference to Example 1, *Plukenetia volubilis* oil fatty acid and sphingosine were reacted to synthesize *Plukenetia volubilis* oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 7.9 min for linolenic acid-sphingosine ceramide, 8.6 min for linoleic acid-sphingosine ceramide, 10.2 min for oleic acid-sphingosine ceramide, 10.4 min for palmitic acid-sphingosine ceramide, 13.6 min for stearic acid-sphingosine ceramide, respectively.

The resulting product was analyzed by high performance liquid chromatography. The contents of linolenic acid-sphingosine ceramide, linoleic acid-sphingosine ceramide, oleic acid-sphingosine ceramide, palmitic acid-sphingosine ceramide, and stearic acid-sphingosine ceramide were 47%, 38%, 6%, 5%, and 1%, respectively, and the rest was other components with trace amounts.

The synthesis of ceramide from *Plukenetia volubilis* oil fatty with phytosphingosine or dihydrosphingosine was carried out with reference to this example.

### Example 12

With reference to Example 10, watermelon seed oil fatty acid and dihydrosphingosine were reacted to synthesize watermelon seed oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 9.5 min for linoleic acid-dihydrosphingosine ceramide, 10.5 min for palmitic acid-dihydrosphingosine ceramide, 11.1 min for oleic acid-dihydrosphingosine ceramide, 13.5 min for stearic acid-dihydrosphingosine ceramide, respectively.

The resulting product was analyzed by high performance liquid chromatography. The contents of linoleic acid-dihydrosphingosine ceramide, palmitic acid-dihydrosphingosine ceramide, oleic acid-dihydrosphingosine ceramide, and stearic acid-dihydrosphingosine ceramide were 66%, 8%, 23%, and 1%, respectively, and the rest was other components with trace amounts.

The synthesis of ceramide from watermelon seed oil fatty acid with phytosphingosine or sphingosine was carried out with reference to this example.

### Example 13

### Synthesis of ceramide from coconut oil fatty acid and phytosphingosine

Coconut oil fatty acid (50 mmol, based on the main fatty acid), EDCI (65 mmol), and Et₃N (65 mmol) were added into a 250 mL round-bottom flask, then 100 mL of dichloromethane was added, followed by stirring at room temperature for 1 hour. Then phytosphingosine (55 mmol) was added to the reaction system, followed by stirring at room temperature, until the reaction was complete by TLC detection.

Post-treatment: Water was added to quench the reaction. The organic layer was separated, dried, filtered, concentrated in vacuum, and then washed with solvent to obtain coconut oil ceramide. The product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 5.7 min for caproic acid-phytosphingosine ceramide, 6.3 min for decanoic acid-phytosphingosine ceramide, 7.5 min for lauric acid-phytosphingosine ceramide, 9.4 min for myristic acid-phytosphingosine ceramide, 10.7 min for palmitic acid-phytosphingosine ceramide, 11.3 min for oleic acid-phytosphingosine ceramide, 13.9 min for stearic acid-phytosphingosine ceramide, respectively.

The resulting product was analyzed by high performance liquid chromatography. The contents of lauric acid-phytosphingosine ceramide, myristic acid-phytosphingosine ceramide, palmitic acid-phytosphingosine ceramide, stearic acid-phytosphingosine ceramide, oleic acid-phytosphingosine ceramide, decanoic acid-phytosphingosine ceramide and caproic acid-phytosphingosine ceramide were 41%, 29%, 12%, 6%, 4%, 1% and 3%, respectively, and the rest was other components with trace amounts.

The synthesis of ceramide from coconut oil fatty acid with sphingosine or dihydrosphingosine is carried out with reference to this example.

### Example 14

With reference to Example 4, *Acer truncatum* seed oil fatty acid and sphingosine were reacted to synthesize *Acer truncatum* seed oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 7.9 min for linolenic acid-sphingosine ceramide, 8.7 min for linoleic acid-sphingosine ceramide, 10.1 min for oleic acid-sphingosine ceramide, 10.4 min for palmitic acid-sphingosine ceramide, 12.7 min for arachidonic acid-sphingosine ceramide, 13.5 min for stearic acid-sphingosine ceramide, 17.8 min for arachidic acid-sphingosine ceramide, respectively.

The resulting product was analyzed by high performance liquid chromatography. The contents of linoleic acid-sphingosine ceramide, oleic acid-sphingosine ceramide, arachidic acid-sphingosine ceramide, arachidonic acid-sphingosine ceramide, linolenic acid-sphingosine ceramide, palmitic acid-sphingosine ceramide, and stearic acid-sphingosine ceramide were 36%, 31%, 9%, 12%, 3%, 2%, and 3%, respectively, and the rest was other components with trace amounts.

The synthesis of ceramide from *Acer truncatum* seed oil fatty acid with phytosphingosine or dihydrosphingosine was carried out with reference to this example.

### Example 15

With reference to Example 4, walnut oil fatty acid and dihydrosphingosine were reacted to synthesize walnut oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for linolenic acid-dihydrosphingosine ceramide, 9.5 min for linoleic acid-dihydrosphingosine ceramide, 10.6 min for palmitic acid-dihydrosphingosine ceramide, 11.1 min for oleic acid-dihydrosphingosine ceramide, respectively.

The resulting product was analyzed by high performance liquid chromatography. The contents of linoleic acid-dihydrosphingosine ceramide, oleic acid-dihydrosphingosine ceramide, palmitic acid-dihydrosphingosine ceramide, and linolenic acid-dihydrosphingosine ceramide were 63%, 23%, 6% and 4%, respectively, and the rest was other components with trace amounts.

The synthesis of ceramide from walnut oil fatty acid with phytosphingosine or sphingosine was carried out with reference to this example.

### Example 16

With reference to Example 1, avocado oil fatty acid and phytosphingosine were reacted to synthesize avocado oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.0 min for palmitoleic acid-phytosphingosine ceramide, 8.3 min for linolenic acid-phytosphingosine ceramide, 9.5 min for linoleic acid-phytosphingosine ceramide, 10.7 min for palmitic acid-phytosphingosine ceramide, 11.3 min for oleic acid-phytosphingosine ceramide, 12.9 min for eicosenoic acid-phytosphingosine ceramide, 13.9 min for stearic acid-phytosphingosine ceramide, respectively.

The resulting product was analyzed by high performance liquid chromatography. The contents of oleic acid-phytosphingosine ceramide, palmitoleic acid-phytosphingosine ceramide, linoleic acid-phytosphingosine ceramide, palmitic acid-phytosphingosine ceramide, stearic acid-phytosphingosine ceramide, linolenic acid-phytosphingosine ceramide, and eicosenoic acid-phytosphingosine ceramide are 70%, 10%, 7%, 8%, 1%, 0.5%, and 0.5%, respectively, and the rest was other components with trace amounts.

The synthesis of ceramide from avocado oil fatty acid with sphingosine or dihydrosphingosine was carried out with reference to this example.

### Example 17

With reference to Example 4, tomato seed oil fatty acid and sphingosine were reacted to synthesize tomato seed oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 7.9 min for linolenic acid-sphingosine ceramide, 8.7 min for linoleic acid-sphingosine ceramide, 10.2 min for oleic acid-sphingosine ceramide, 10.4 min for palmitic acid-sphingosine ceramide, 13.6 min for stearic acid-sphingosine ceramide, 17.8 min for arachidic acid-sphingosine ceramide, respectively.

The resulting product was analyzed by high performance liquid chromatography. The contents of linoleic acid-sphingosine ceramide, oleic acid-sphingosine ceramide, palmitic acid-sphingosine ceramide, stearic acid-sphingosine ceramide, linolenic acid-sphingosine ceramide, and arachidic acid-sphingosine ceramide were 74%, 13%, 5%, 2%, 2%, and 1%, respectively, and the rest was other components with trace amounts.

The synthesis of ceramide from tomato seed oil fatty acid with phytosphingosine or dihydrosphingosine was carried out with reference to this example.

### Example 18

With reference to Example 2, *Ximenia americana* seed oil fatty acid and dihydrosphingosine were reacted to synthesize *Ximenia americana* seed oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.3 min for linolenic acid-dihydrosphingosine ceramide, 11.1 min for oleic acid-dihydrosphingosine ceramide, 13.5 min for stearic acid-dihydrosphingosine ceramide, 17.2 min for ximenynic acid-dihydrosphingosine ceramide, 21.8 min for behenic acid-dihydrosphingosine ceramide, and 24.1 min for nervonic acid-dihydrosphingosine ceramide, respectively.

The resulting product was analyzed by high performance liquid chromatography. The contents of oleic acid-dihydrosphingosine ceramide, linolenic acid-dihydrosphingosine ceramide, stearic acid-dihydrosphingosine ceramide, behenic acid-dihydrosphingosine ceramide, ximenynic acid-dihydrosphingosine ceramide, and nervonic acid-dihydrosphingosine ceramide were 50%, 31%, 3%, 2%, 8%, and 4%, respectively, and the rest was other components with trace amounts.

The synthesis of ceramide from *Ximenia americana* seed oil fatty acid with phytosphingosine or sphingosine was carried out with reference to this example.

### Example 19

### Synthesis of ceramide from Prinsepia utilis oil fatty acid and phytosphingosine

Step 1: 50 g of *Prinsepia utilis* oil was dissolved in 80 mL of tetrahydrofuran, cooled in an ice bath, and then 100 mL of potassium hydroxide (25 wt.%) solution was added dropwise. After the addition was complete, it was heated to room temperature for reaction, until the reaction was complete by TLC detection.

Post-treatment: Dilute hydrochloric acid (3N) was added to adjust the pH value of the reaction system to 3. 120 mL of ethyl acetate was added to extract the aqueous phase. 100 mL of saturated brine was added to wash once, and then anhydrous Na₂SO₄ was added to dry the organic phase, then filtered and concentrated in vacuum to obtain 40.6 g *Prinsepia utilis* oil fatty acid.

Step 2: *Prinsepia utilis* oil fatty acid (50 mmol, based on the main fatty acid), EDCI (55 mmol), and DMAP (55 mmol) were added into a 250 mL round-bottom flask, then 100 mL of dichloromethane was added, followed by stirring at room temperature for 1 hour. Then phytosphingosine (55 mmol) was added to the reaction system, following by stirring at room temperature, until the reaction was complete by TLC detection.

Post-treatment: Water was added to quench the reaction. The organic layer was separated, dried, filtered, concentrated in vacuum, and then washed with solvent to obtain *Prinsepia utilis* oil ceramide. The product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 9.5 min for linoleic acid-phytosphingosine ceramide, 10.7 min for palmitic acid-phytosphingosine ceramide, 11.3 min for oleic acid-phytosphingosine ceramide, 13.9 min for stearic acid-phytosphingosine ceramide, 16.5 min for arachidic acid-phytosphingosine ceramide, respectively.

The resulting product was analyzed by high performance liquid chromatography. The contents of linoleic acid-phytosphingosine ceramide, oleic acid-phytosphingosine ceramide, palmitic acid-phytosphingosine ceramide, stearic acid-phytosphingosine ceramide, and arachidic acid-phytosphingosine ceramide were 39%, 36%, 12%, 9%, and 1%, respectively, and the rest was other components with trace amounts.

The synthesis of ceramide from *Prinsepia utilis* oil fatty acid with sphingosine or dihydrosphingosine was carried out with reference to this example.

### Example 20

With reference to Example 4, safflower seed oil fatty acid and sphingosine were reacted to synthesize safflower seed oil ceramide, and the product was analyzed by HPLC. Chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 7.9 min for linolenic acid-sphingosine ceramide, 8.7 min for linoleic acid-sphingosine ceramide, 10.2 min for oleic acid-sphingosine ceramide, 10.4 min for palmitic acid-sphingosine ceramide, 13.5 min for stearic acid-sphingosine ceramide, 17.7 min for arachidic acid-sphingosine ceramide, respectively.

The resulting product was analyzed by high performance liquid chromatography. The contents of linoleic acid-sphingosine ceramide, oleic acid-sphingosine ceramide, palmitic acid-sphingosine ceramide, linolenic acid-sphingosine ceramide, stearic acid-sphingosine ceramide, and arachidic acid-sphingosine ceramide were 72%, 17%, 7%, 0.5%, 1%, and 0.5%, respectively, and the rest was other components with trace amounts.

The synthesis of ceramide from safflower seed oil fatty acid with phytosphingosine or dihydrosphingosine was carried out with reference to this embodiment.

### Example 21

With reference to Example 3, peony seed oil fatty acids and dihydrosphingosine were reacted to synthesize peony seed oil ceramide, and the product was analyzed by HPLC under the following chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.3 min for α-linolenic acid-dihydrosphingosine ceramide, 9.5 min for linoleic acid-dihydrosphingosine ceramide, 10.6 min for palmitic acid-dihydrosphingosine ceramide, 11.2 min for oleic acid-dihydrosphingosine ceramide, and 13.5 min for stearic acid-dihydrosphingosine ceramide.

The resulting product was analyzed by high performance liquid chromatography (HPLC), which revealed that the contents of α-linolenic acid-dihydrosphingosine ceramide, linoleic acid-dihydrosphingosine ceramide, oleic acid-dihydrosphingosine ceramide, palmitic acid-dihydrosphingosine ceramide, and stearic acid-dihydrosphingosine ceramide were respectively 43%, 34%, 16%, 1%, 3%, and the rest was other ingredients in trace amounts.

The synthesis of ceramides from peony seed oil fatty acids with phytosphingosine or sphingosine is carried out with reference to this Example.

### Example 22

With reference to Example 4, sunflower oil fatty acids and phytosphingosine were reacted to synthesize sunflower oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 9.4 min for linoleic acid-phytosphingosine ceramide, 10.7 min for palmitic acid-phytosphingosine ceramide, 11.3 min for oleic acid-phytosphingosine ceramide, 13.9 min for stearic acid-phytosphingosine ceramide, 16.5 min for arachidic acid-phytosphingosine ceramide, and 18.3min for behenic acid-phytosphingosine ceramide.

The resulting product was analyzed by high performance liquid chromatography (HPLC), which revealed that the contents of linoleic acid-phytosphingosine ceramide, oleic acid-phytosphingosine ceramide, stearic acid-phytosphingosine ceramide, palmitic acid-phytosphingosine ceramide, behenic acid-phytosphingosine ceramide, arachidic acid-phytosphingosine ceramide were respectively 58%, 27%, 4%, 5%, 2%, 1%, and the rest was other ingredients in trace amounts.

The synthesis of ceramides from sunflower oil fatty acids with sphingosine or dihydrosphingosine was performed analogously to this example.

### Example 23

With reference to Example 1, perilla seed oil fatty acids and sphingosine were reacted to synthesize perilla seed oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 7.9 min for α-linolenic acid-sphingosylceramide, 8.7 min for linoleic acid-sphingosylceramide, 10.2 min for oleic acid-sphingosylceramide, 10.4 min for palmitic acid-sphingosylceramide, and 13.6 min for stearic acid-sphingosylceramide.

The resulting product was analyzed by high performance liquid chromatography (HPLC), which revealed that the contents of α-linolenic acid-sphingosylceramide, linoleic acid-sphingosylceramide, oleic acid-sphingosylceramide, stearic acid-sphingosylceramide, palmitic acid-sphingosylceramide were respectively 70%, 15%, 7%, 2%, 2%, and the rest was other ingredients in trace amounts.

The synthesis of ceramides from perilla seed oil fatty acids with phytosphingosine or dihydrosphingosine was performed analogously to this example.

### Example 24

With reference to Example 3, linseed oil fatty acids and dihydrosphingosine were reacted to synthesize linseed oil ceramide, and the products were analyzed by HPLC under the following chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.3 min for linolenic acid-dihydrosphingosine ceramide, 9.5 min for linoleic acid-dihydrosphingosine ceramide, 10.7 min for palmitic acid-dihydrosphingosine ceramide, 11.1 min for oleic acid-dihydrosphingosine ceramide, and 13.6 min for stearic acid-dihydrosphingosine ceramide.

The resulting product was analyzed by high performance liquid chromatography (HPLC), which revealed that the contents of linolenic acid-dihydrosphingosine ceramide, linoleic acid-dihydrosphingosine ceramide, oleic acid-dihydrosphingosine ceramide, palmitic acid-dihydrosphingosine ceramide, and stearic acid-dihydrosphingosine ceramide were respectively 55%, 15%, 11%, 8%, 7%, and the rest was other ingredients in trace amounts.

The synthesis of ceramides from linseed oil fatty acids with phytosphingosine or sphingosine is carried out with reference to this Example.

### Example 25

With reference to Example 4, *Silybum marianum* oil fatty acids and phytosphingosine were reacted to synthesize *Silybum marianum* oil ceramide, and the product was analyzed by HPLC under the following chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.2 min for linolenic acid-phytosphingosine ceramide, 9.5 min for linoleic acid-phytosphingosine ceramide, 10.7 min for palmitic acid-phytosphingosine ceramide, 11.3 min for oleic acid-phytosphingosine ceramide, 13.9 min for stearic acid-phytosphingosine ceramide, 16.5 min for arachidic acid-phytosphingosine ceramide, and 18.3 min for behenic acid-phytosphingosine ceramide.

The resulting product was analyzed by high performance liquid chromatography, which revealed that the contents of linoleic acid-phytosphingosine ceramide, oleic acid-phytosphingosine ceramide, palmitic acid-phytosphingosine ceramide, stearic acid-phytosphingosine ceramide, arachidic acid-phytosphingosine ceramide, behenic acid-phytosphingosine ceramide, and linolenic acid-phytosphingosine ceramide were respectively 65%, 18%, 5%, 3%, 3%, 2%, 1%, and the rest was other ingredients in trace amounts.

The synthesis of ceramides from *Silybum marianum* oil fatty acids with sphingosine or dihydrosphingosine was performed analogously to this example.

### Example 26

With reference to Example 4, shea butter fatty acids and sphingosine were reacted to synthesize shea butter ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.7 min for linoleic acid-sphingosylceramide, 10.2 min for oleic acid-sphingosylceramide, 10.5 min for palmitic acid-sphingosylceramide, 13.6 min for stearic acid-sphingosylceramide and 17.8 min for arachidic acid-sphingosylceramide.

The resulting product was analyzed by high performance liquid chromatography (HPLC), which revealed that the contents of oleic acid-sphingosine ceramide, stearic acid-sphingosine ceramide, linoleic acid-sphingosine ceramide, palmitic acid-sphingosine ceramide, and arachidic acid-sphingosine ceramide were respectively 37%, 48%, 3%, 7%, 2%, and the rest was other ingredients in trace amounts.

The synthesis of ceramides from shea butter fatty acids with phytosphingosine or dihydrosphingosine was performed analogously to this example.

### Example 27

With reference to Example 4, evening primrose oil fatty acids and dihydrosphingosine were reacted to synthesize evening primrose oil ceramide, and the product was analyzed by HPLC under the following chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were 8.2 min for γ-linolenic acid-dihydrosphingosine ceramide, 9.5 min for linoleic acid-dihydrosphingosine ceramide, 10.6 min for palmitic acid-dihydrosphingosine ceramide, 11.0 min for oleic acid-dihydrosphingosine ceramide, 13.5 min for stearic acid-dihydrosphingosine ceramide, and 14.0 min for arachidonic acid-dihydrosphingosine ceramide 14.0 min.

The resulting product was analyzed by HPLC, which revealed that the contents of linoleic acid-dihydrosphingosine ceramide, oleic acid-dihydrosphingosine ceramide, γ-linolenic acid-dihydrosphingosine ceramide, palmitic acid-dihydrosphingosine ceramide, stearic acid-dihydrosphingosine ceramide, and arachidonoic acid-dihydrosphingosine ceramide were respectively 65%, 16%, 10%, 2%, 2%, 1%, and the rest was other ingredients in trace amounts.

The synthesis of ceramides from evening primrose oil fatty acids with phytosphingosine or sphingosine is carried out with reference to this Example.

### Example 28

With reference to Example 1, Argan fatty acids and phytosphingosine were reacted to synthesize argan ceramide, and the products were analyzed by HPLC under the following chromatographic conditions: a Shimadzu High Performance Liquid Chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.3 min for linolenic acid-phytosphingosine ceramide, 9.5 min for linoleic acid-phytosphingosine ceramide, 10.7 min for palmitic acid-phytosphingosine ceramide, 11.3 min for oleic acid-phytosphingosine ceramide, 12.9 min for arachidonic acid-phytosphingosine ceramide, 13.9 min for stearic acid-phytosphingosine ceramide, 16.5 min for arachidonic acid-phytosphingosine ceramide, and 18.3 min for behenic acid-phytosphingosine ceramide.

The resulting product was analyzed by high performance liquid chromatography, which revealed that the contents of oleic acid-phytosphingosine ceramide, linoleic acid-phytosphingosine ceramide, palmitic acid-phytosphingosine ceramide, stearic acid-phytosphingosine ceramide, arachidic acid-phytosphingosine ceramide, linolenic acid-phytosphingosine ceramide, behenic acid-phytosphingosine ceramide and arachidonoic acid-phytosphingosine ceramide were respectively 50%, 33%, 5%, 6%, 0.5%, 0.5%, 0.5%, 0.5%, 0.5%, 0.5%, and the rest was other ingredients in trace amounts.

The synthesis of ceramides from argan fatty acids with sphingosine or dihydrosphingosine was performed analogously to this example.

### Example 29

With reference to Example 2, cannabis seed oil fatty acids and sphingosine were synthesized from cannabis seed oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 7.9 min for linolenic acid-sphingosylceramide, 8.7 min for linoleic acid-sphingosylceramide, 10.2 min for oleic acid-sphingosylceramide, 10.4 min for palmitic acid-sphingosylceramide, and 13.5 min for stearic acid-sphingosylceramide.

The resulting product was analyzed by high performance liquid chromatography (HPLC), which revealed that the contents of linoleic acid-sphingosylceramide, linolenic acid-sphingosylceramide, oleic acid-sphingosylceramide, palmitic acid-sphingosylceramide, and stearic acid-sphingosylceramide were respectively 69%, 10%, 7%, 8%, 3.5%, and the rest was other ingredients in trace amounts.

The synthesis of ceramides from cannabis seed oil fatty acids with phytosphingosine or dihydrosphingosine was performed analogously to this example.

### Example 30

With reference to Example 3, Baiji flower seed oil fatty acids and phytosphingosine were reacted to synthesize Baiji flower seed oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 2% water+98% methanol.

The HPLC retention times of the components were respectively 11.3 min for oleic acid-phytosphingosine ceramide, 22.6 min for cis-5-eicosatetraenoic acid-phytosphingosine ceramide, 23.8 min for cis-5,13-docosadienoic acid-phytosphingosine ceramide, 28.8 min for erucic acid-phytosphingosine ceramide, and 32.6 min for cis-5-docosatetraenoic acid-phytosphingosine ceramide.

The resulting product was analyzed by high performance liquid chromatography (HPLC), which revealed that the contents of cis-5-eicosatetraenoic acid-phytosphingosine ceramide, cis-5,13-docosadienoic acid-phytosphingosine ceramide, erucic acid-phytosphingosine ceramide, cis-5-docosatetraenoic acid-phytosphingosine ceramide, and oleic acid-phytosphingosine ceramide were respectively 55%, 21%, 15%, 4%, 1%, and the rest was other ingredients in trace amounts.

The synthesis of ceramides from the fatty acids of meadowfoam seed oil with sphingosine or dihydrosphingosine was performed analogously to this example.

### Example 31

With reference to Example 3, baobab seed oil fatty acids and dihydrosphingosine were reacted to synthesize baobab seed oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.2 min for linolenic acid-dihydrosphingosine ceramide, 8.5 min for myristic acid-dihydrosphingosine ceramide, 8.9 min for palmitoleic acid-dihydrosphingosine ceramide, 9.5 min for linoleic acid-dihydrosphingosine ceramide, 10.7 min for palmitic acid-dihydrosphingosine ceramide, 11.1 min for oleic acid-dihydrosphingosine ceramide, 13.5 min for stearic acid-dihydrosphingosine ceramide, and 16.0 min for arachidonic acid-dihydrosphingosine ceramide.

The resulting product was analyzed by HPLC, which revealed that the contents of oleic acid-dihydrosphingosine ceramide, linoleic acid-dihydrosphingosine ceramide, palmitic acid-dihydrosphingosine ceramide, stearic acid-dihydrosphingosine ceramide, linolenic acid-dihydrosphingosine ceramide, arachidic acid-dihydrosphingosine ceramide, palmitoleic acid-dihydrosphingosine ceramide, myristic acid-dihydrosphingosine ceramide were respectively 46%, 17%, 26%, 5%, 2%, 1%, 0.5%, 0.5%, and the rest was other ingredients in trace amounts.

The synthesis of ceramides from baobab seed oil fatty acids with phytosphingosine or sphingosine is carried out with reference to this Example.

### Example 32

With reference to Example 19, cactus seed oil fatty acids and sphingosine were reacted to synthesize cactus seed oil ceramide, and the product was analyzed by HPLC under the following chromatographic conditions: a Shimadzu high performance liquid chromatograph (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 7.9 min for linolenic acid-sphingosylceramide, 8.2 min for palmitoleic acid-sphingosylceramide, 10.2 min for oleic acid-sphingosylceramide, 10.5 min for palmitic acid-sphingosylceramide, 13.6 min for stearic acid-sphingosylceramide, and 17.8 min for arachidonic acid-sphingosylceramide.

The resulting product was analyzed by high performance liquid chromatography (HPLC), which revealed that the contents of linolenic acid-sphingosylceramide, oleic acid-sphingosylceramide, palmitoleic acid-sphingosylceramide, palmitic acid-sphingosylceramide, stearic acid-sphingosylceramide, arachidonic acid-sphingosylceramide were respectively 50%, 24%, 13%, 6%, 4.5%, 1%, and the rest was other ingredients in trace amounts.

The synthesis of ceramides from cactus seed oil fatty acids with phytosphingosine or dihydrosphingosine was performed analogously to this example.

### Example 33

With reference to Example 3, wheat germ oil fatty acids and dihydrosphingosine were synthesized from wheat germ oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.3 min for linolenic acid-dihydrosphingosine ceramide, 9.5 min for linoleic acid-dihydrosphingosine ceramide, 10.6 min for palmitic acid-dihydrosphingosine ceramide, 11.1 min for oleic acid-dihydrosphingosine ceramide, 13.5 min for stearic acid-dihydrosphingosine ceramide, 14.0 min for arachidonic acid-dihydrosphingosine ceramide, and 16.0 min for arachidonic acid-dihydrosphingosine ceramide.

The resulting product was analyzed by high performance liquid chromatography (HPLC), which revealed that the contents of linoleic acid-dihydrosphingosine ceramide, oleic acid-dihydrosphingosine ceramide, palmitic acid-dihydrosphingosine ceramide, linolenic acid-dihydrosphingosine ceramide, arachidonic acid-dihydrosphingosine ceramide, stearic acid-dihydrosphingosine ceramide, arachidonic acid-dihydrosphingosine ceramide, were respectively 53%, 32%, 8%, 2.5%, 1%, 0.5%, 0.5%, and the rest was other ingredients in trace amounts.

The synthesis of ceramides from wheat germ oil fatty acids with phytosphingosine or sphingosine is carried out with reference to this Example.

### Example 34

With reference to Example 2, *Malania oleifera* oil fatty acids and phytosphingosine were reacted to synthesize *Malania oleifera* oil ceramide, and the products were analyzed by HPLC, HPLC chromatographic conditions: using a Shimadzu high performance liquid chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 9.5 min for linoleic acid-phytosphingosine ceramide, 10.7 min for palmitic acid-phytosphingosine ceramide, 11.3 min for oleic acid-phytosphingosine ceramide, 16.5 min for arachidonic acid-phytosphingosine ceramide, and 22.2 min for ceramic acid-phytosphingosine ceramide.

The resulting product was analyzed by high performance liquid chromatography (HPLC), which revealed that the contents of neuronic acid-phytosphingosine ceramide, oleic acid-phytosphingosine ceramide, arachidonic acid-phytosphingosine ceramide, palmitic acid-phytosphingosine ceramide and linoleic acid-phytosphingosine ceramide were respectively 52%, 35%, 8%, 2%, 1%, and the rest was other ingredients in trace amounts.

The synthesis of ceramides from fatty acids of *Malania oleifera* oil with sphingosine or dihydrosphingosine was performed analogously to this example.

### Example 35

With reference to Example 2, blackcurrant seed oil fatty acids and dihydrosphingosine were reacted to synthesize blackcurrant seed oil ceramide, and the products were analyzed by HPLC, HPLC chromatographic conditions: using a Shimadzu HPLC (LC-2030C 3D Plus) with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 100% methanol.

The HPLC retention times of the components were respectively 8.3 min for linolenic acid-dihydrosphingosine ceramide, 9.5 min for linoleic acid-dihydrosphingosine ceramide, 10.6 min for palmitic acid-dihydrosphingosine ceramide, 11.1 min for oleic acid-dihydrosphingosine ceramide, 13.5 min for stearic acid-dihydrosphingosine ceramide, and 16.0 min for arachidonic acid-dihydrosphingosine ceramide.

The resulting product was analyzed by HPLC, which revealed that the contents of linoleic acid-dihydrosphingosine ceramide, linolenic acid-dihydrosphingosine ceramide, oleic acid-dihydrosphingosine ceramide, palmitic acid-dihydrosphingosine ceramide, stearic acid-dihydrosphingosine ceramide, and arachidic acid-dihydrosphingosine ceramide, were respectively 45%, 31%, 13%, 5%, 2%, 2%, and the rest was other ingredients in trace amounts.

The synthesis of ceramides from blackcurrant seed oil fatty acids with phytosphingosine or sphingosine is carried out with reference to this Example.

### Example 36

With reference to Example 2, DHA algal oil fatty acids and sphingosine were reacted to synthesize DHA algal oil ceramide, and the product was analyzed by HPLC under the following chromatographic conditions: a Shimadzu High Performance Liquid Chromatograph (LC-2030C 3D Plus), with an Innoval ODS-2 column (4.6 × 250 mm, 5 µm) at a column temperature of 30°C, injection volume of 10 µL, flow rate of 1.0 mL/min, evaporation temperature of 40°C, carrier gas flow rate of 2.5 L/min, and mobile phase of 2% water+98% methanol.

The HPLC retention times of the components were respectively 7.9 min for linolenic acid-sphingosylceramide, 10.2 min for oleic acid-sphingosylceramide, 10.5 min for palmitic acid-sphingosylceramide, 13.6 min for stearic acid-sphingosylceramide, 15.6 min for EPA-sphingosylceramide, 17.8 min for arachidonic acid-sphingosylceramide, 19.1 min for DHA- sphingosine ceramide, and 24.0 min for behenic acid-sphingosine ceramide.

The resulting product was analyzed by HPLC, which revealed that the contents of DHA-sphingosylceramide, oleic acid-sphingosylceramide, palmitic acid-sphingosylceramide, stearic acid-sphingosylceramide, linolenic acid-sphingosylceramide, EPA-sphingosylceramide, arachidonic acid-sphingosylceramide, and behenic acid-sphingosylceramide were respectively 62%, 23%, 10%, 1%, 1%, 0.5%, 0.5%, 0.5%, 0.5%, and the rest was other ingredients in trace amounts.

The synthesis of ceramides from DHA algal oil fatty acids with phytosphingosine or dihydrosphingosine was performed analogously to this example.

The samples in Examples 37 to 43 all are plant-derived ceramides synthesized by reacting the corresponding plant oil fatty acids with phytosphingosine. For instance, cottonseed oil ceramide are derived from the reaction of cottonseed oil fatty acids with phytosphingosine.

### Example 37

### MTT assay for detecting the dffects of compounds on cell proliferation viability

HaCaT cells were seeded into 96-well plates at a density of 1×10⁴ cells per well and cultured overnight in an incubator. After 24 h, the supernatant was removed, and 100 µL of medium containing samples at varying concentrations was added. Following 24 h of incubation, the medium was discarded, and 100 µL of thiazolyl blue (MTT) was added to each well. Absorbance at 450 nm was measured, and cell viability was calculated as: Cell viability (%) = (A_{treated} wells / A_{blank} wells) × 100%.

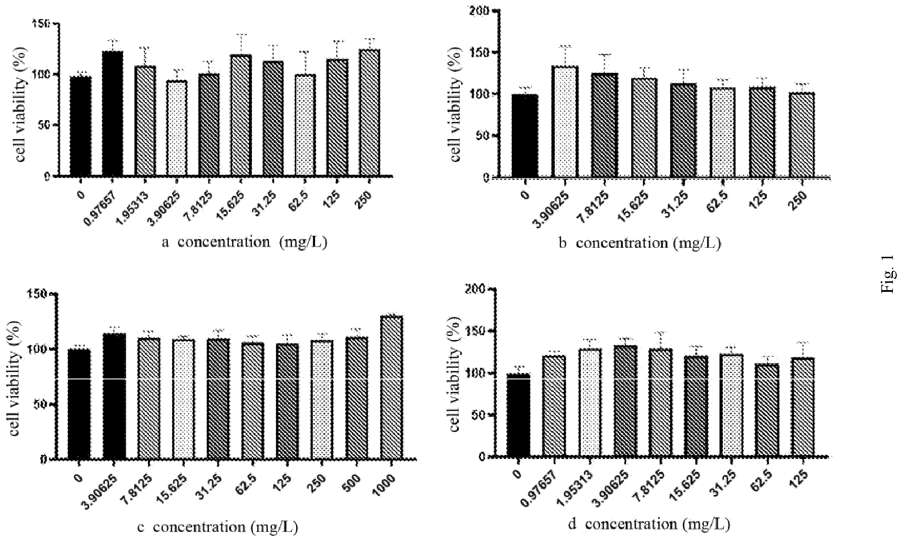

As shown in Figure 1, cottonseed oil ceramide, camellia oil ceramide, *Silybum marianum* oil ceramide, and perilla seed oil ceramide exhibited enhancements in cell viability with a stable concentration gradient, demonstrating obvious effects in promoting cell proliferation and good tissue repairing ability. Among them, for cottonseed oil ceramide (Figure 1a), at concentrations of 0.97657, 1.95313, 3.90625, 7.8125, 15.625, 31.25, 62.5, 125, and 250 mg/L, cell viabilities were 122.63%, 108.38%, 94.33%, 100.86%, 119.66%, 113.59%, 100.40%, 115.47%, and 124.87%, respectively, and the effective concentration was as low as 1 mg/L; for camellia oil ceramide (Figure 1b), at concentrations of 3.90625, 7.8125, 15.625, 31.25, 62.5, 125, and 250 mg/L, cell viabilities were 133.79%, 125.07%, 120.00%, 112.92%, 108.17%, 108.34%, and 102.18%, respectively, and the effective concentration was as low as 4 mg/L, with a safe concentration of 250 mg/L; for *Silybum marianum* oil ceramide (Figure 1c), at concentrations of 3.90625, 7.8125, 15.625, 31.25, 62.5, 125, 250, 500, and 1000 mg/L, cell viabilities were 114.17%, 110.45%, 109.29%, 109.76%, 105.92%, 105.23%, 108.13%, 111.03%, and 130.31%, respectively, and the effective concentration was as low as 4 mg/L, with a safe concentration of 1000 mg/L; for perilla seed oil ceramide (Figure 1d), at concentrations of 0.97657, 1.95313, 3.90625, 7.8125, 15.625, 31.25, 62.5, and 125 mg/L, cell viabilities were 121.42%, 129.10%, 132.97%, 129.16%, 120.44%, 122.89%, 111.23%, and 118.53%, respectively, and the effective concentration was as low as 4 mg/L, with a safe concentration up to 1000 mg/L.

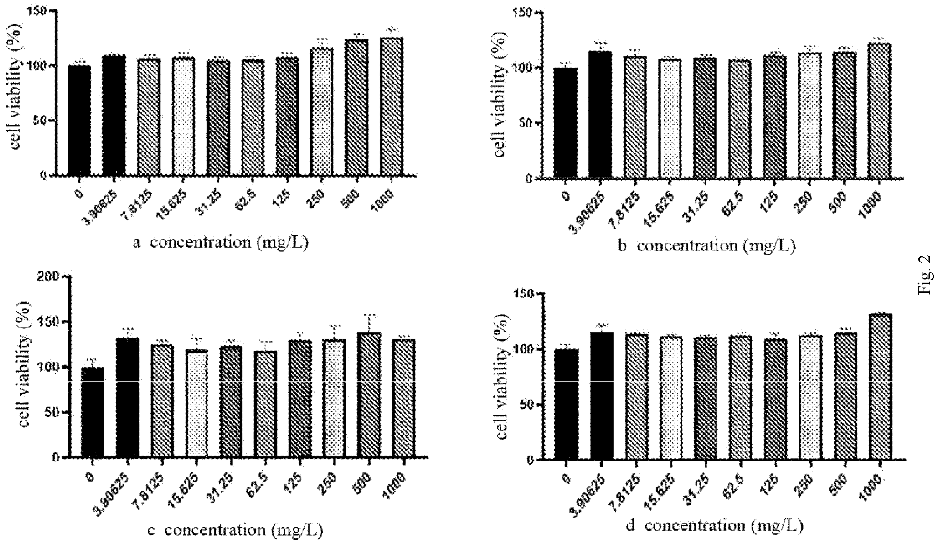

As shown in Figure 2, peony seed oil ceramide, borage oil ceramide, *Ximenia americana* seed oil ceramide, and *Plukenetia volubilis* oil ceramide exhibited enhancements in cell viability with a stable concentration gradient, demonstrating obvious effects in promoting cell proliferation and good tissue repairing ability. Among them, for peony seed oil ceramide (Figure 2a), at concentrations of 3.90625, 7.8125, 15.625, 31.25, 62.5, 125, 250, 500, and 1000 mg/L, cell viabilities were 109.76%, 106.50%, 107.43%, 105.11%, 105.34%, 107.55%, 116.61%, 124.04%, and 125.96%, respectively, and the effective concentration was as low as 4 mg/L, with a safe concentration as high as 1000 mg/L; for borage oil ceramide (Figure 2b), at concentrations of 3.90625, 7.8125, 15.625, 31.25, 62.5, 125, 250, 500, and 1000 mg/L, the cell viabilities were 115.13%, 110.87%, 108.06%, 108.89%, 107.02%, 111.49%, 114.09%, 114.61%, and 122.52%, respectively, and the effective concentration was as low as 4 mg/L, with a safe concentrations as high as 1,000 mg/L; for *Ximenia americana* seed oil ceramide (Figure 2c), at concentrations of 3.90625, 7.8125, 15.625, 31.25, 62.5, 125, 250, 500, and 1000 mg/L, the cell viabilities were 132.61%, 124.80%, 118.90%, 123.14%, 117.91%, 129.79%, 130.79%, 138.51%, and 131.20%, respectively, and the effective concentration was as low as 4 mg/L, with a safe concentration of 1000 mg/L; for *Plukenetia volubilis* oil ceramide (Figure 2d), at concentrations of 3.90625, 7.8125, 15.625, 31.25, 62.5, 125, 250, 500, and 1000 mg/L, the cell viabilities were 115.55%, 113.88%, 111.80%, 111.39%, 112.32%, 109.52%, 113.05%, 115.03%, and 131.98%, respectively, and the effective concentration was as low as 4mg/L, with a safe concentration as high as 1000 mg/L.

Ceramide 2 was tested for its effects on cell proliferation viability using the same method. At concentrations of 3.90625, 7.8125, 15.625, 31.25, 62.5, 125, 250, 500, and 1000 mg/L, the cell viabilities were 61.49%, 60.03%, 55.41%, 54.64%, 53.37%, 46.95%, 44.05%, 40.35%, and 39.42%, respectively. Ceramide 2 exhibited inhibitory effects on cell proliferation and demonstrated inferior tissue repair potential compared to plant-derived ceramides.

### Example 38

### Evaluation of skin barrier repair effects by cell migration assay

Principle: When cells grow to form a confluent monolayer, a scratch tool is used to create a cell-free blank area on the confluent monolayer cells, the cells in the blank area are removed by a mechanical force, and through a period of incubation, the migration of the cells to the cell-free area is observed, and the migration ability of the cells is reflected by measuring the migration distance of the cells.

### Procedure:

1. Marking the culture plate. Use a marker pen and ruler to draw evenly spaced horizontal lines (approximately 0.5-1 cm apart) on the back of a 6-well plate, ensuring at least 5 lines cross each well. Avoid thick lines.
2. Cell seeding. Seed approximately 5 × 10⁵cells per well (the number varies for varied cells, adjusting based on cell growth rate), and the principle of inoculation is to achieve 100% confluence overnight.
3. Cell scratching. On the following day, use a pipette tip perpendicular to the cell layer to create scratches along the pre-marked lines on the back of the plate (It is best to use the same tip for all wells).
4. Cell washing. After the scratching was completed, rinse the cells three times with sterile PBS to remove dislodged cells, leaving a clear visible scratch gap, and then replace with fresh serum-free medium.
5. Cell culturing and observation. The samples were diluted with culture medium (For *Silybum marianum* oil ceramide, perilla seed oil ceramide, peony seed oil ceramide, *Ximenia americana* seed oil ceramide, borage oil ceramide, *Plukenetia volubilis* oil ceramide, the concentration was 5mg/L; for camellia oil ceramide, safflower seed oil ceramide, cottonseed oil ceramide, watermelon seed oil ceramide, *Acer truncatum* seed oil ceramide, coconut oil ceramide, the concentration was 20mg/L; and the concentration of ceramide 3B was 100mg/L) and added to the cell culture dish, and the cells were incubated in the incubator at 37 °C with 5wt% CO₂, and the cells were taken out after 24h, the width of the scratches was observed and measured under a microscope, photographed, and then healing rates were calculated by the software Image J.

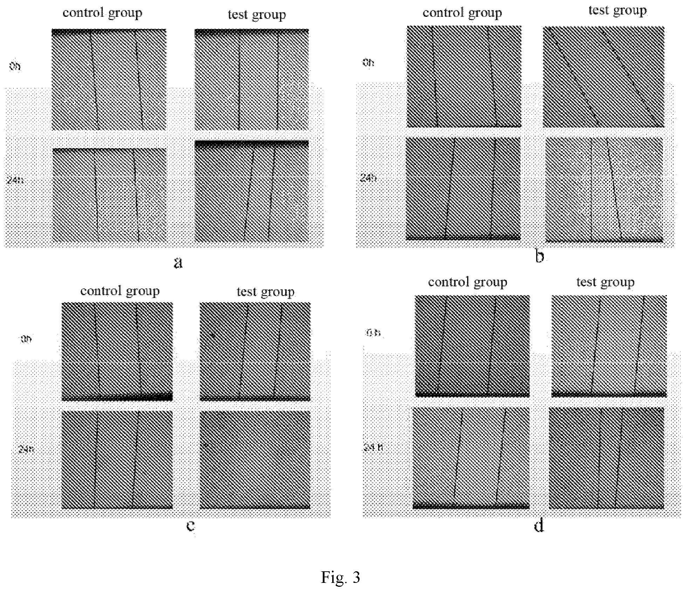

As shown in Figure 3, compared to the solvent control group, test groups showed narrower scratch widths, indicating superior tissue repair ability for *Silybum marianum* oil ceramide, perilla seed oil ceramide, peony seed oil ceramide, and *Ximenia americana* seed oil ceramide. The healing rate of the solvent control group was 27.33% after 24h, the healing rate of *Silybum marianum* oil ceramide (Figure 3a) was 90.23% after 24h, and the healing rate of ceramide 3B was 59.32% after 24h. The healing rate of the solvent control group was 48.35% after 24 h, the healing rate of perilla seed oil ceramide (Figure 3b) was 92.41% after 24 h, the healing rate of ceramide 3B was 59.32% after 24 h. The healing rate of the solvent control group was 32.58% after 24h, the healing rate of peony seed oil ceramide (Figure 3c) was 93.21% after 24h, and the healing rate of ceramide 3B was 59.32% after 24h. The healing rate of the solvent control group was 29.58% after 24h, the healing rate of *Ximenia americana* seed oil ceramide (Figure 3d) was 94.26% after 24h, and the healing rate of ceramide 3B was 59.32% after 24h.

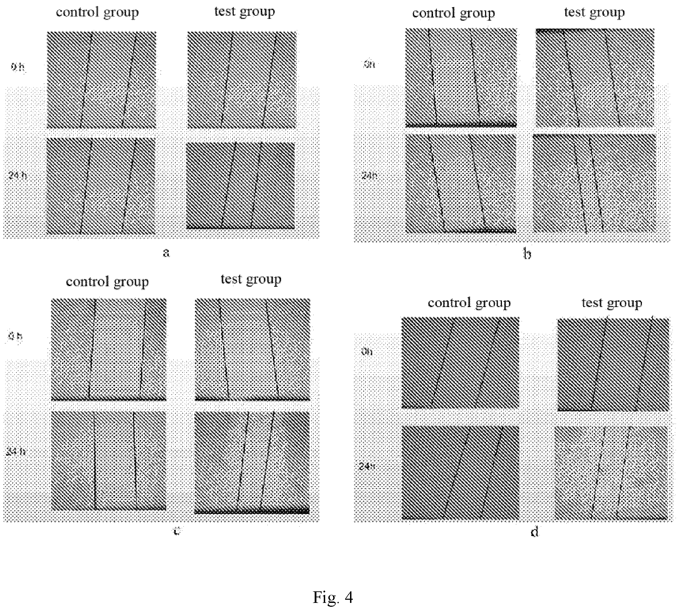

As shown in Figure 4, compared to the solvent control group, test groups showed narrower scratch widths, indicating superior tissue repair ability for borage oil ceramide, *Plukenetia volubilis* oil ceramide, camellia oil ceramide, safflower seed oil ceramide. The healing rate of the solvent control group was 29.58% after 24h, the healing rate of borage oil ceramide (Figure 4a) was 82.31% after 24h, and the healing rate of ceramide 3B was 59.32% after 24h. The healing rate of the solvent control group was 29.58% after 24h, the healing rate of *Plukenetia volubilis* oil ceramide (Figure 4b) was 92.71% after 24h, and the healing rate of ceramide 3B was 59.32% after 24h. The healing rate of the solvent control group was 34.25% after 24h, the healing rate of camellia oil ceramide (Figure 4c) was 88.75% after 24h, and the healing rate of ceramide 3B was 59.32% after 24h. The healing rate of the solvent control group was 41.25% after 24h, the healing rate of safflower seed oil ceramide (Figure 4d) was 94.25% after 24h, and the healing rate of ceramide 3B was 59.32% at 24h.

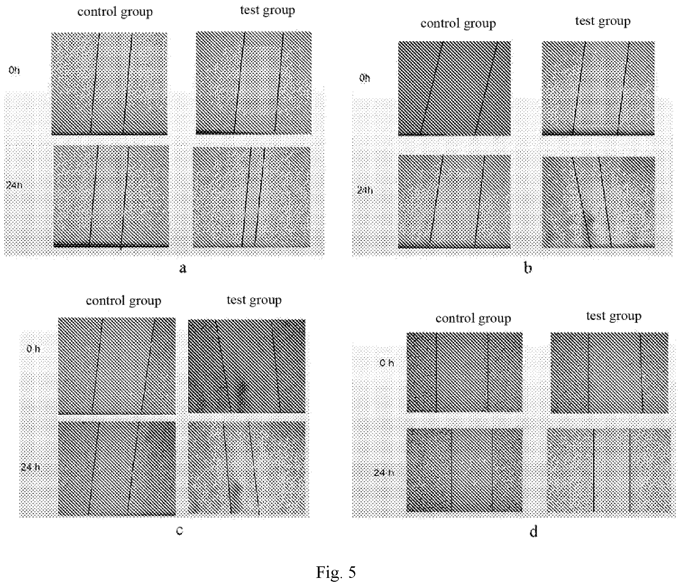

As shown in Figure 5, compared to the solvent control group, test groups showed narrower scratch widths, indicating superior tissue repair ability for cottonseed oil ceramide, watermelon seed oil ceramide, *Acer truncatum* seed oil ceramide, and coconut oil ceramide. The healing rate of the solvent control group was 38.22% after 24h, the healing rate of cottonseed oil ceramide (Figure 5a) was 96.21% after 24h, and the healing rate of ceramide 3B was 59.32% after 24h. The healing rate of the solvent control group was 35.21% after 24h, the healing rate of watermelon seed oil ceramide (Figure 5b) was 88.53% after 24h, and the healing rate of ceramide 3B was 59.32% after 24h. The healing rate of the solvent control group was 31.38% after 24h, the healing rate of *Acer truncatum* seed oil ceramide (Figure 5c) was 87.22% after 24h, and the healing rate of ceramide 3B was 59.32%. The healing rate of the solvent control group was 29.58% after 24h, the healing rate of coconut oil ceramide (Figure 5d) was 82.31% after 24h, and the healing rate of ceramide 3B was 59.32% after 24h.

Thus, the novel compounds significantly enhanced cell migration and healing rates, demonstrating robust skin tissue repair activity and superior efficacy compared to Ceramide 3B.

### Example 39

### Evaluation of anti-aging effects by elastase inhibition assay

Elastase inhibition method: Add 2 mL of 2 mg/mL elastase solution to samples of varying concentrations, vortex-mix thoroughly, and incubate at 37°C with shaking at 400 rpm for 20 min. Immediately add 5 mL of pH 6.0 phosphate buffer (0.5 mol/L), vortex and mix well, take an appropriate amount of mixing solution to a 2 mL centrifuge tube, centrifuge at 9,391 × g for 10 min. Then carefully pipette 200 µL of the supernatant into a 96-well plate and measure the absorbance at a wavelength of 495 nm with a microplate reader, and perform spectral scanning from 400-800 nm.

The substrate + enzyme solution was used as the blank control group, the substrate + enzyme + sample solution was used as the enzyme inhibition group, and the substrate + sample without enzyme solution was used as the background subtraction group. Each group had three replicate wells. Inhibition rate (%)=[1-(An -An')/(A0 -A0')]×100%, where A0 is the absorbance of adding enzyme (no sample) A0' is the absorbance of adding substrate only (no sample or enzyme), An is the absorbance of adding sample solution only, An' is the absorbance of adding sample (no enzyme). If An'>An, then it shows promotion ability, promotion rate (%) = [1-(An' -An)/(A0 -A0')] × 100%.

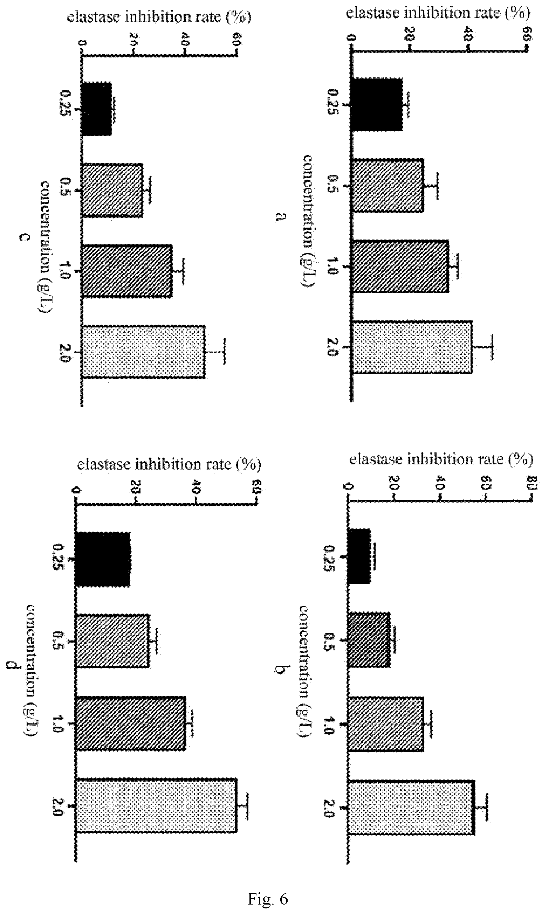

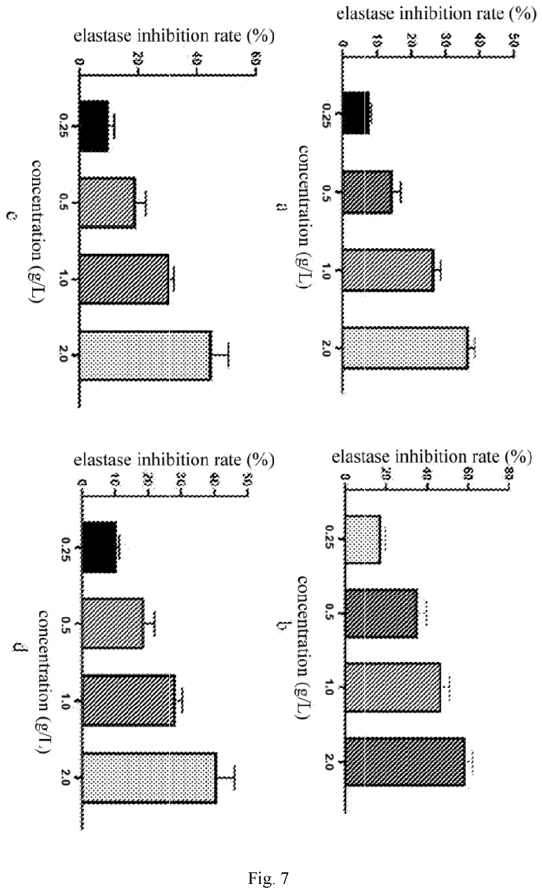

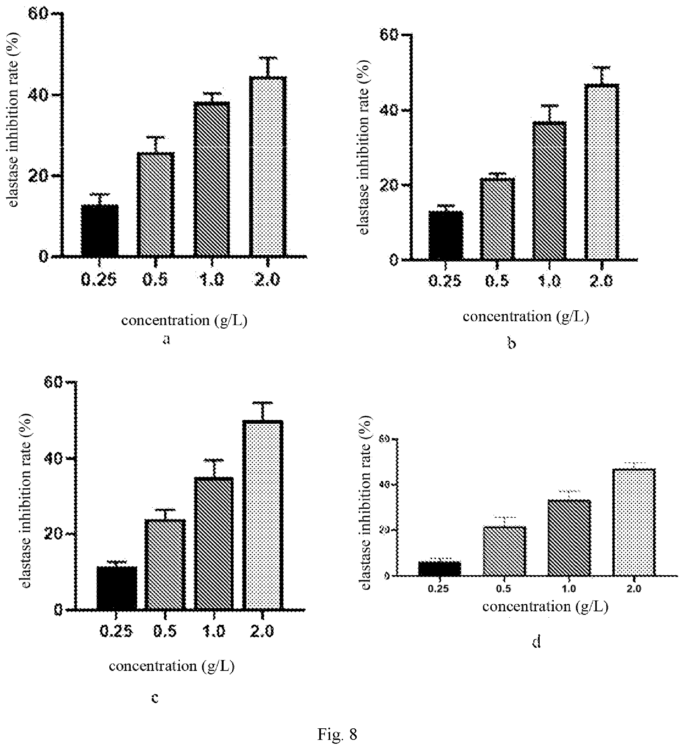

The results are shown in FIGS. 6-8 and the table below. the novel compounds demonstrated significant elastase-inhibitory activity across tested concentrations. Ceramide 2 exhibited inferior inhibitory effects compared to plant-derived ceramides at equivalent concentrations.

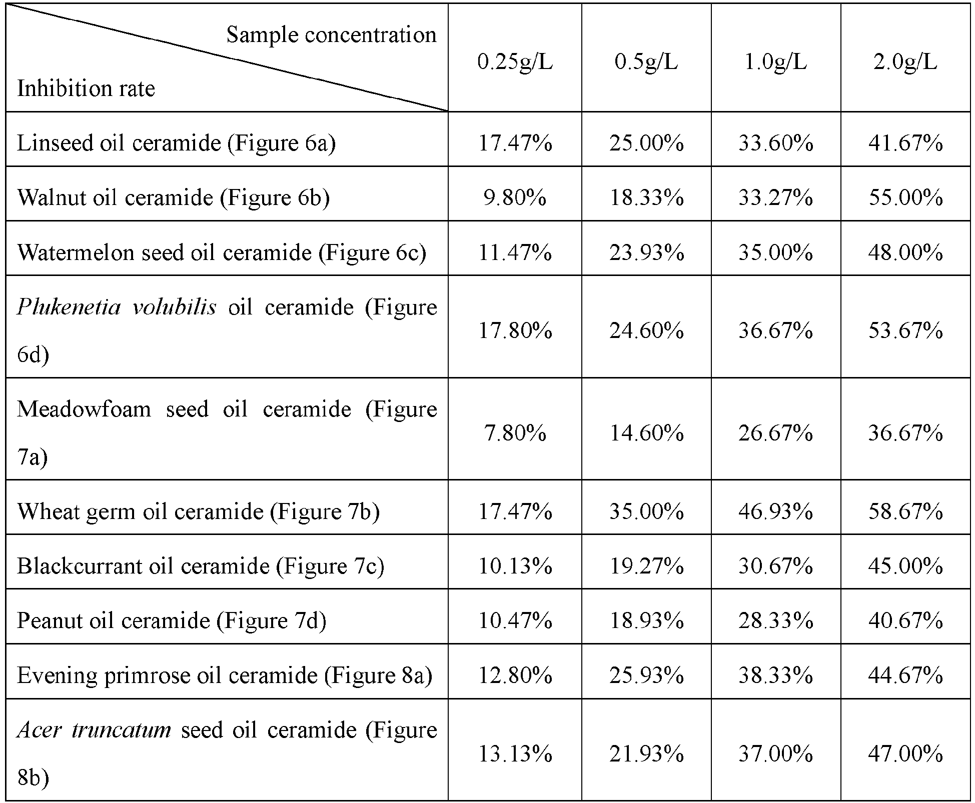

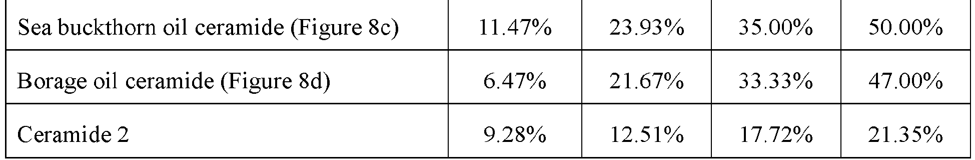

### Example 40

### Evaluation of anti-inflammatory and repair effects via LPS-induced cell model

B16 mouse melanoma cells were seeded in 96-well plates at a density of 1 × 10⁴cells/well, placed in the incubator overnight for adhesion. After 24 h, the supernatant was discarded, and 100 µL of samples diluted in DMEM medium at varying concentrations was added, and the negative control group was DMEM medium without samples. Each group had three replicate wells and incubated at 37°C with 5wt.% CO₂. After 2 h of administration, 10 µg/mL LPS was added to the lipopolysaccharide model group and test groups, followed by continued incubation for 24 h. After the reaction was over, 50 µL of cell supernatant was collected, and IL-6 gene expression in the cells was detected by using an IL-6 ELISA kit.

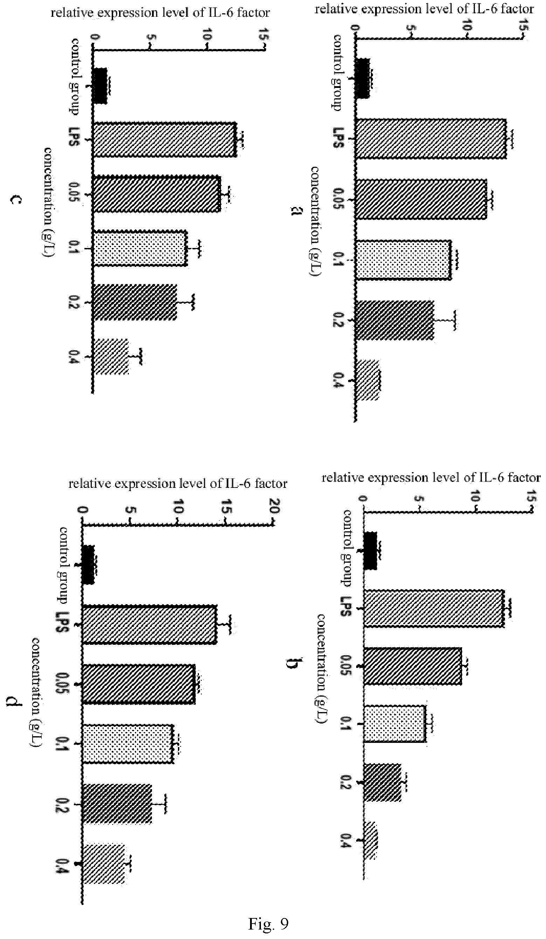

As shown in Figure 9a, under the stimulation of LPS at a working concentration of 10 µg/mL, the IL-6 level was 10.95-fold of the basal level, and treatment with rapeseed oil ceramide at concentrations of 50 mg/L, 100 mg/L, 200 mg/L, and 400 mg/L significantly reduced the IL-6 levels to 0.87-fold, 0.64-fold, 0.52-fold, and 0.16-fold of the LPS model group, respectively, demonstrating a dose-dependent effect; as shown in Figure 9b, under the stimulation of LPS at a working concentration of 10 µg/mL, the IL-6 level was 10.16-fold of the basal level, and treatment with *Silybum marianum* oil ceramide at concentrations of 50 mg/L, 100 mg/L, 200 mg/L, and 400 mg/L significantly reduced the IL-6 levels to 0.70-fold, 0.45-fold, 0.27-fold, and 0.09-fold of the LPS model group, respectively, demonstrating a dose-dependent effect; as shown in Figure 9c, under the stimulation of LPS at a working concentration of 10 µg/mL, the IL-6 level was 10.16-fold of the basal level, and treatment with perilla seed oil ceramide at concentrations of 50 mg/L, 100 mg/L, 200 mg/L, and 400 mg/L significantly reduced the IL-6 levels to 0.89-fold, 0.66-fold, 0.59-fold, and 0.25-fold of the LPS model group, demonstrating a dose-dependent effect; as shown in Figure 9d, under the stimulation of LPS at a working concentration of 10 µg/mL, the IL-6 level was 11.51-fold of the basal level, and treatment with sunflower oil ceramide at concentrations of 50 mg/L, 100 mg/L, 200 mg/L, and 400 mg/L significantly reduced the IL-6 levels to 0.84-fold, 0.68-fold, 0.52-fold, and 0.31-fold of the LPS model group, respectively, demonstrating a dose-dependent effect.

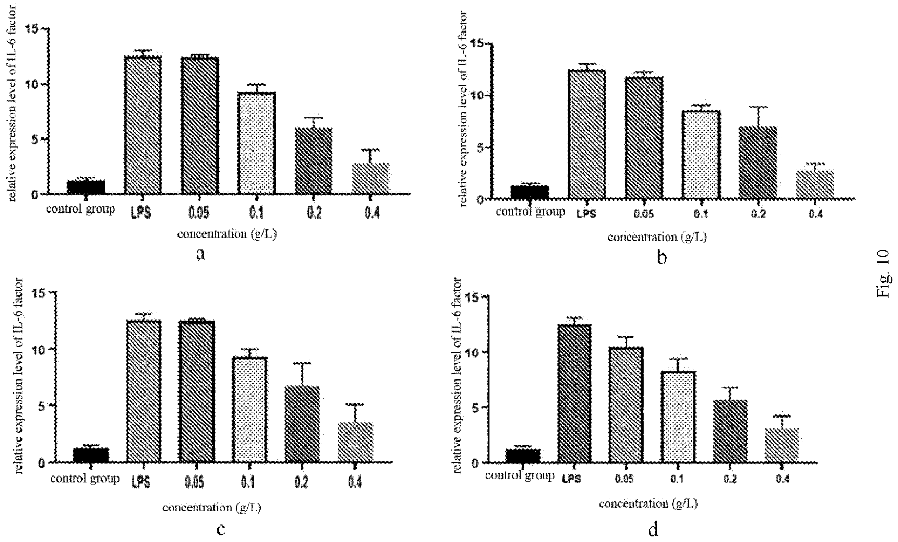

As shown in Figure 10a, under the stimulation of LPS with a working concentration of 10 µg/mL, the IL-6 level was 10.16-fold of the basal level, and treatment with peony seed oil ceramide at concentrations of 50 mg/L, 100 mg/L, 200 mg/L, and 400 mg/L significantly reduced the IL-6 levels to 0.99-fold, 0.74-fold, 0.48-fold, and 0.22-fold of the LPS model group, respectively, demonstrating a dose-dependent effect; as shown in Figure 10b, under the stimulation of LPS at a working concentration of 10 µg/mL, the IL-6 level was 10.16-fold of the basal level, and treatment with safflower seed oil ceramide at concentrations of 50 mg/L, 100 mg/L, 200 mg/L, and 400 mg/L significantly reduced the IL-6 levels to 0.94-fold, 0.69-fold, 0.56-fold, and 0.22-fold of the LPS model group, respectively, demonstrating a dose-dependent effect; as shown in Figure 10c, under the stimulation of LPS at a working concentration of 10 µg/mL, the IL-6 level was 10.16-fold of the basal level, and treatment with safflower seed oil ceramide at concentrations of 50 mg/L, 100 mg/L, 200 mg/L, and 400 mg/L significantly reduced the IL-6 levels to 0.99-fold, 0.74-fold, 0.53-fold, and 0.27-fold of the LPS model group, respectively, demonstrating a dose-dependent effect; as shown in Figure 10d, under the stimulation of LPS at a working concentration of 10 µg/mL, the IL-6 level was 10.16-fold of the basal level, and treatment with walnut oil ceramide at concentrations of 50 mg/L, 100 mg/L, 200 mg/L, and 400 mg/L significantly reduced the IL-6 levels to 0.84-fold, 0.66-fold, 0.45-fold, and 0.25-fold of the LPS model group, respectively, demonstrating a dose-dependent effect.

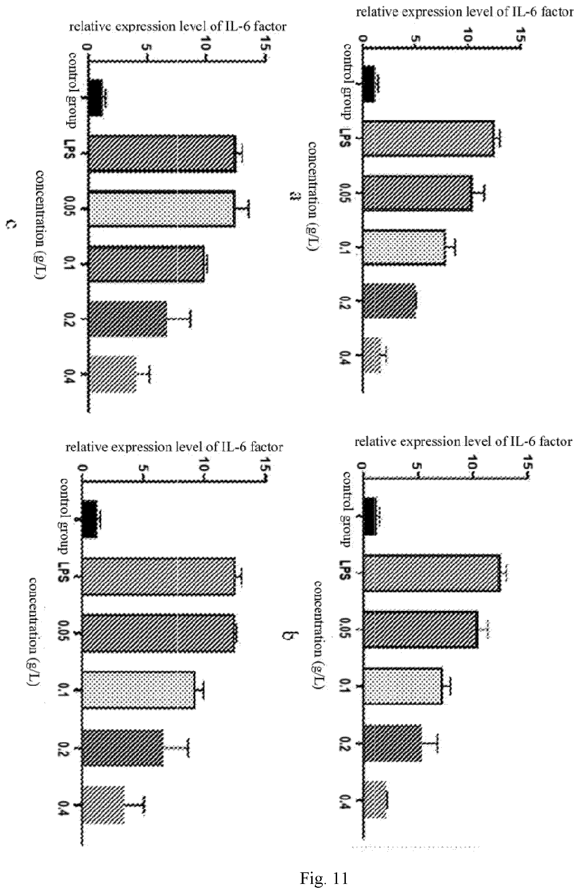

As shown in Figure 11a, under the stimulation of LPS at a working concentration of 10 µg/mL, the IL-6 level was 10.16-fold of the basal level, and treatment with *Plukenetia volubilis* ceramide at concentrations of 50 mg/L, 100 mg/L, 200 mg/L, and 400 mg/L significantly reduced the IL-6 levels to 0.84-fold, 0.63-fold, 0.40-fold, and 0.14-fold of the LPS model group, respectively, demonstrating a dose-dependent effect; as shown in Figure 11b, under the stimulation of LPS at a working concentration of 10 µg/mL, the IL-6 level was 10.16-fold of the basal level, and treatment with borage oil ceramide at concentrations of 50 mg/L, 100 mg/L, 200 mg/L, and 400 mg/L significantly reduced the IL-6 levels to 0.84-fold, 0.58-fold, 0.43-fold, and 0.17-fold of the LPS model group, respectively, demonstrating a dose-dependent effect; as shown in Figure 11c, under the stimulation of LPS at a working concentration of 10 µg/mL, the IL-6 level was 10.16-fold of the basal level, and treatment with grape seed oil ceramide at concentrations of 50 mg/L, 100 mg/L, 200 mg/L, and 400 mg/L significantly reduced the IL-6 levels to 0.99-fold, 0.79-fold, 0.53-fold, and 0.33-fold of the LPS model group, respectively, demonstrating a dose-dependent effect; as shown in Figure 11d, under the stimulation of LPS at a working concentration of 10 µg/mL, the IL-6 level was 10.16-fold of the basal level, and treatment of cactus seed oil ceramide at concentrations of 50 mg/L, 100 mg/L, 200 mg/L, and 400 mg/L significantly reduced the IL-6 levels to 0.99-fold, 0.74-fold, 0.53-fold, and 0.27-fold of the LPS model group, respectively, demonstrating a dose-dependent effect.

Therefore, the plant-derived ceramides of the present invention exhibit potent anti-inflammatory effects and promote the repair of inflammation-damaged skin.

### Example 41

MMP1, also known as interstitial collagenase or matrix metalloproteinase, belongs to the matrix metalloproteinase family. Its primary substrates are fibrillar collagens, and it degrades collagen fibers and gelatin in the extracellular matrix, altering the cellular microenvironment. MMP1 plays a critical role in elastin metabolism. Inhibition of MMP1 enhances collagen and elastin synthesis in fibroblasts, while reduced MMP activity accelerates collagen production.

HaCaT cells were seeded in 96-well plates at a density of 1×10⁵cells/well in an incubator and incubated overnight. After 24 h, the supernatant was discarded, and 100 µL of medium containing test samples at varying concentrations was added. The model group received no sample, and the negative control group was DMEM medium without samples. Each group had three replicate wells and incubated at 37°C with 5 wt.% CO₂ for 2 h, and then irradiated with UVA or UVB radiation. The distance between the UV radiation source and the cells was 15 cm, the UVA intensity was 200 mJ/cm² with a radiation time of 2 h, and the UVB intensity was 50 mJ/cm² with a radiation time of 1 h. After the radiation was over, the incubation was continued in the incubator for 12 h. Then MMP-1 gene expression in the cells was detected by using an MMP-1 ELISA kit. Inhibition rate=1-(MMP1 expression in test group/MMP1 expression in model group)× 100%.

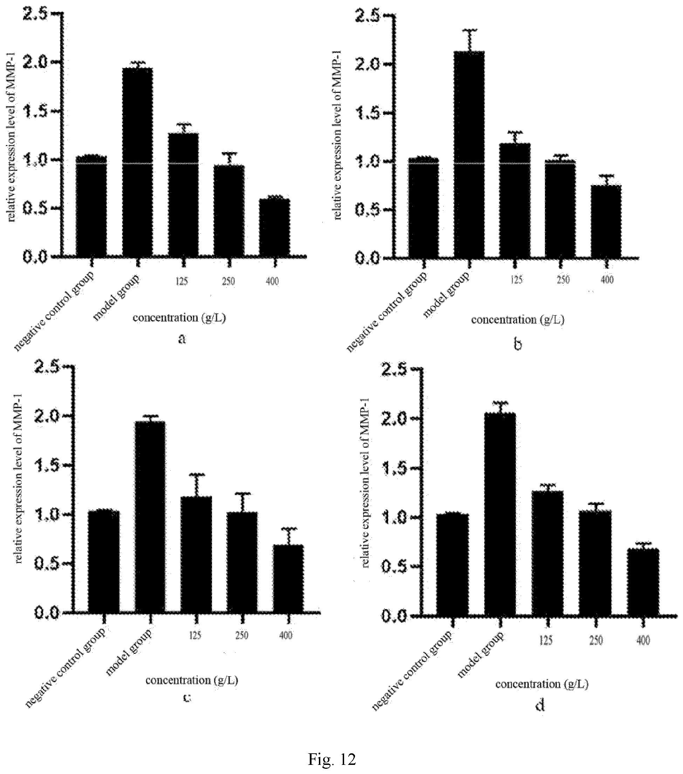

As shown in Figure 12, for UVA, the MMP1 expression level of the negative control group was set as 1, the expression level of the model group was 1.90, and *Silybum marianum* oil ceramide (Figure 12a) at concentrations of 125 mg/L, 250 mg/L, and 400 mg/L exhibited inhibition rates of 34%, 51%, and 69%, respectively, on MMP1 expression relative to the model group; for UVB, the MMP1 expression level of the negative control group was set as 1, the expression level of the model group was 2.33, and *Silybum marianum* oil ceramide (Figure 12b) at concentrations of 125 mg/L, 250 mg/L, and 400 mg/L exhibited inhibition rates of 44%, 52%, and 65%, respectively, on MMP1 expression relative to the model group. For UVA, the MMP1 expression level of the negative control group was set as 1, the expression level of the model group was 1.90, and linseed oil ceramide (Figure 12c) at concentrations of 125 mg/L, 250 mg/L, and 400 mg/L exhibited inhibition rates of 39%, 47%, and 64%, respectively, on MMP1 expression relative to the model group; for UVB, the MMP1 expression level of the negative control group was set as 1, the expression level of the model group was 2.33, and linseed oil ceramide (Figure 12d) at concentrations of 125 mg/L, 250 mg/L, and 400 mg/L exhibited inhibition rates of 38%, 48%, and 67%, respectively, on MMP1 expression relative to the model group.

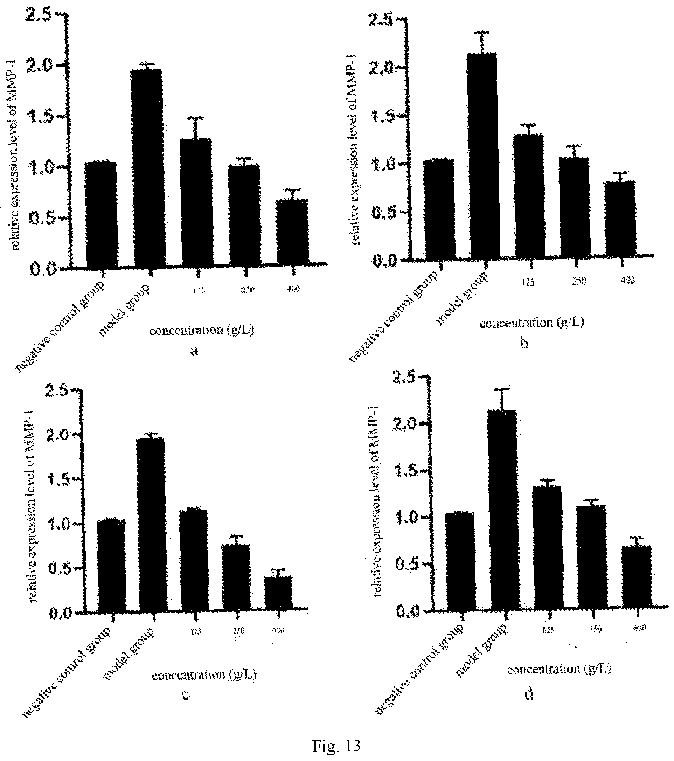

As shown in Figure 13, for UVA, the MMP1 expression level of the negative control group was set as 1, the expression level of the model group was 1.90, and peony seed oil ceramide (Figure 13a) at concentrations of 125 mg/L, 250 mg/L, and 400 mg/L exhibited inhibition rates of 35%, 49%, and 67%, respectively, on MMP1 expression relative to the model group; for UVB, the MMP1 expression level of the negative control group was set as 1, the expression level of the model group was 2.33, and peony seed oil ceramide (Figure 13b) at concentrations of 125 mg/L, 250 mg/L, and 400 mg/L exhibited inhibition rates of 40%, 51%, and 63%, respectively, on MMP1 expression relative to the model group. For UVA, the MMP1 expression level of the negative control group was set as 1, the expression level of the model group was 1.90, and tomato seed oil ceramide (Figure 13c) at concentrations of 125 mg/L, 250 mg/L, and 400 mg/L exhibited inhibition rates of 42%, 62%, and 81%, respectively, on MMP1 expression relative to the model group; for UVB, the MMP1 expression level of the negative control group was set as 1, the expression level of the model group was 2.33, and tomato seed oil ceramide (Figure 13d) at concentrations of 125 mg/L, 250 mg/L, and 400 mg/L exhibited inhibition rates of 39%, 49%, and 69%, respectively, on MMP1 expression relative to the model group.

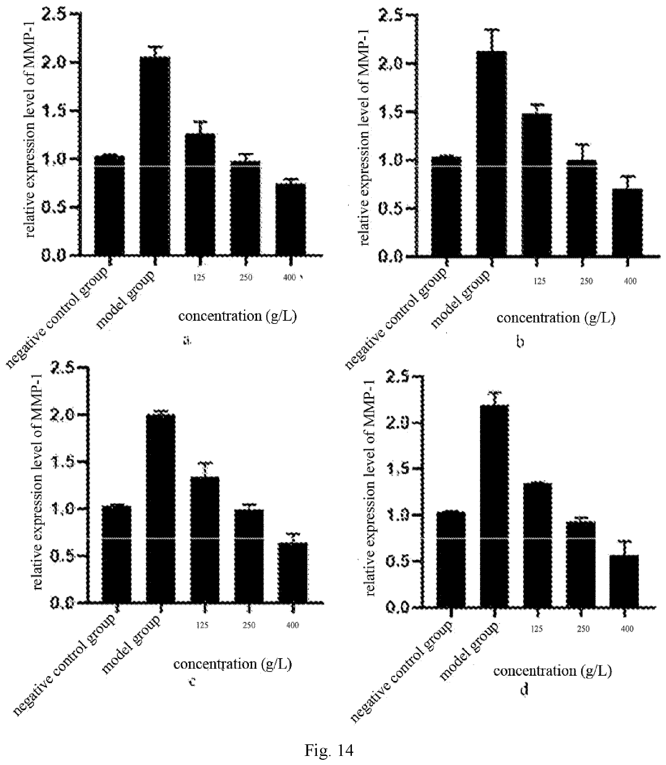

As shown in Figure 14, for UVA, the MMP1 expression level of the negative control group was set as 1, the expression level of the model group was 1.90, and *Acer truncatum* oil ceramide (Figure 14a) at concentrations of 125 mg/L, 250 mg/L, and 400 mg/L exhibited inhibition rates of 35%, 50%, and 62%, respectively, on MMP1 expression relative to the model group; for UVB, the MMP1 expression level of the negative control group was set as 1, the expression level of the model group was 2.33, and *Acer truncatum* oil ceramide (Figure 14b) at concentrations of 125 mg/L, 250 mg/L, and 400 mg/L exhibited inhibition rates of 30%, 53%, and 67%, respectively, on MMP1 expression relative to the model group. For UVA, the MMP1 expression level of the negative control group was set as 1, the expression level of the model group was 1.90, and coconut oil ceramide (Figure 14c) at concentrations of 125 mg/L, 250 mg/L, and 400 mg/L exhibited inhibition rates of 31%, 49%, and 67%, respectively, on MMP1 expression relative to the model group; for UVB, the MMP1 expression level of the negative control group was set as 1, the expression level of the model group was 2.33, and coconut oil ceramide (Figure 14d) at concentrations of 125 mg/L, 250 mg/L, and 400 mg/L exhibited inhibition rates of 38%, 58%, and 74%, respectively, on MMP1 expression relative to the model group.

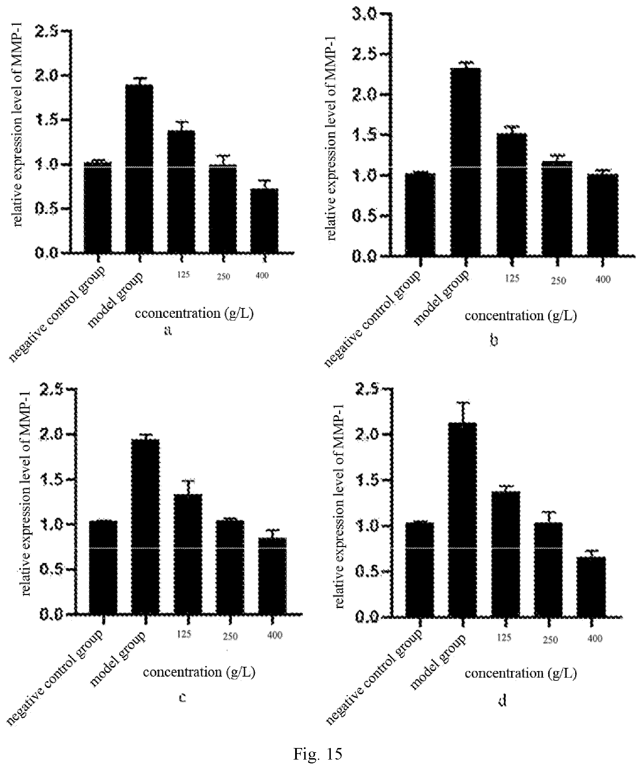

As shown in Figure 15, for UVA, the MMP1 expression level of the negative control group was set as 1, the expression level of the model group was 1.90, and cottonseed oil (Figure 15a) at concentrations of 125 mg/L, 250 mg/L, and 400 mg/L exhibited inhibition rates of 27%, 48%, and 62%, respectively, on MMP1 expression relative to the model group; for UVB, the MMP1 expression level of the negative control group was set as 1, the expression level of the model group was 2.33, and cottonseed oil (Figure 15b) at concentrations of 125 mg/L, 250 mg/L, and 400 mg/L exhibited inhibition rates of 35%, 49%, and 56%, respectively, on MMP1 expression relative to the model group. For UVA, the MMP1 expression level of the negative control group was set as 1, the expression level of the model group was 1.90, and sea buckthorn oil ceramide (Figure 15c) at concentrations of 125 mg/L, 250 mg/L, and 400 mg/L exhibited inhibition rates of 31%, 46%, and 56%, respectively, on MMP1 expression relative to the model group; for UVB, the MMP1 expression level of the negative control group was set as 1, the expression level of the model group was 2.33, and sea buckthorn oil ceramide (Figure 15d) at concentrations of 125 mg/L, 250 mg/L, and 400 mg/L exhibited inhibition rates of 35%, 51%, and 69%, respectively, on MMP1 expression relative to the model group.

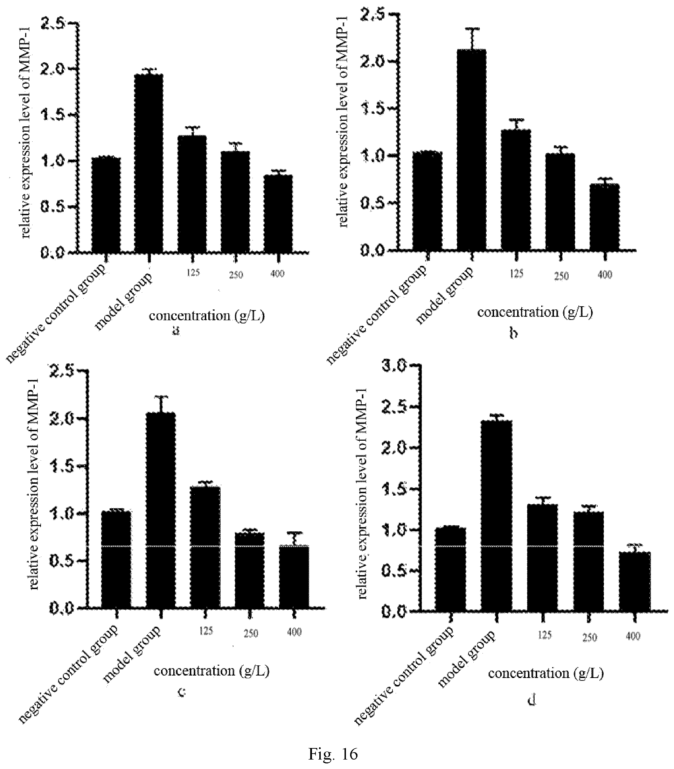

As shown in Figure 16, for UVA, the MMP1 expression level of the negative control group was set as 1, the expression level of the model group was 1.90, and argan oil ceramide (Figure 16a) at concentrations of 125 mg/L, 250 mg/L, and 400 mg/L exhibited inhibition rates of 34%, 44%, and 56%, respectively, on MMP1 expression relative to the model group; for UVB, the MMP1 expression level of the negative control group was set as 1, the expression level of the model group was 2.33, and argan oil ceramide (Figure 16b) at concentrations of 125 mg/L, 250 mg/L, and 400 mg/L exhibited inhibition rates of 40%, 52%, and 67%, respectively, on MMP1 expression relative to the model group. For UVA, the MMP1 expression level of the negative control group was set as 1, the expression level of the model group was 1.90, and wheat germ oil ceramide (Figure 16c) at concentrations of 125 mg/L, 250 mg/L, and 400 mg/L exhibited inhibition rates of 38%, 61%, and 67%, respectively, on MMP1 expression relative to the model group; for UVB, the MMP1 expression level of the negative control group was set as 1, the expression level of the model group was 2.33, and wheat germ oil ceramide (Figure 16d) at concentrations of 125 mg/L, 250 mg/L, and 400 mg/L exhibited inhibition rates of 43%, 48%, and 68%, respectively, on MMP1 expression relative to the model group.

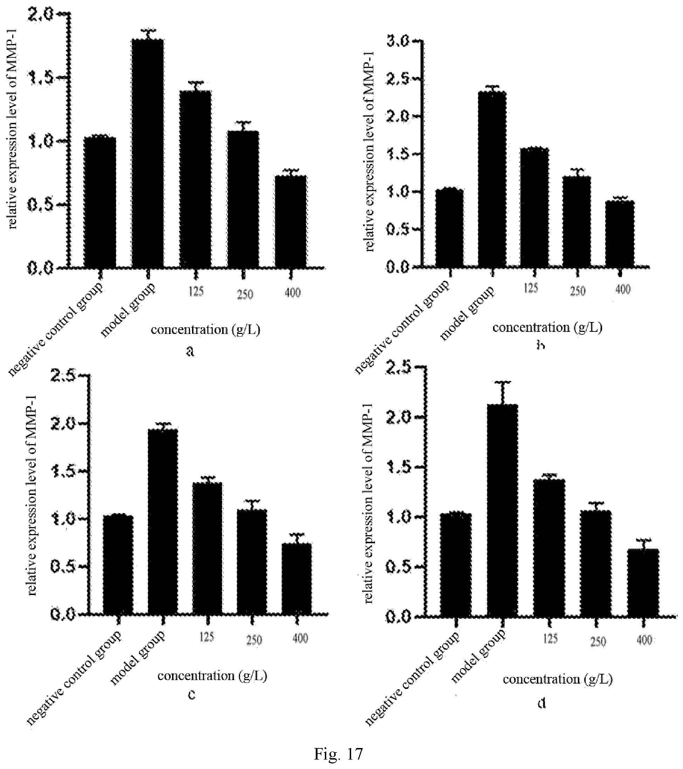

As shown in Figure 17, for UVA, the MMP1 expression level of the negative control group was set as 1, the expression level of the model group was 1.90, and European plum seed oil ceramide (Figure 17a) at concentrations of 125 mg/L, 250 mg/L, and 400 mg/L exhibited inhibition rates of 38%, 51%, and 67%, respectively, on MMP1 expression relative to the model group; for UVB, the MMP1 expression level of the negative control group was set as 1, the expression level of the model group was 2.33, and European plum seed oil ceramide (Figure 17b) at concentrations of 125 mg/L, 250 mg/L, and 400 mg/L exhibited inhibition rates of 43%, 48%, and 68%, respectively, on MMP1 expression relative to the model group. For UVA, the MMP1 expression level of the negative control group was set as 1, the expression level of the model group was 1.90, and hazelnut oil ceramide (Figure 17c) at concentrations of 125 mg/L, 250 mg/L, and 400 mg/L exhibited inhibition rates of 29%, 44%, and 62%, respectively, on MMP1 expression relative to the model group; for UVB, the MMP1 expression level of the negative control group was set as 1, the expression level of the model group was 2.33, and hazelnut oil ceramide (Figure 17d) at concentrations of 125 mg/L, 250 mg/L, and 400 mg/L exhibited inhibition rates of 35%, 50%, and 68%, respectively, on MMP1 expression relative to the model group.

After UVA irradiation, keratinocytes promote increased MMP1 expression in fibroblasts, leading to degradation of the skin extracellular matrix and collagen, thereby causing skin photoaging. The above results indicate that plant-drived ceramides can inhibit UV-induced MMP1 production in fibroblasts, demonstrating potential efficacy in preventing skin photoaging.

### Example 42

### DPPH free radical scavenging assay for antioxidant activity

DPPH is 1,1-diphenyl-2- picrylhydrazyl and can be used in antioxidant experiments.

Samples at corresponding concentrations (50, 100, 200, 400, 800 mg/L) were mixed with 0.1 mol/L DPPH and absolute ethanol at a 1:1 volume ratio, respectively. DPPH and absolute ethanol were mixed at a 1:1 volume ratio, reacted at room temperature in the dark for 30 min, and the absorbance was measured at 517 nm. The absorbance of the sample mixed with DPPH is denoted as A1, the absorbance of the sample mixed with absolute ethanol is denoted as A2, and the absorbance of DPPH mixed with absolute ethanol is denoted as A3. The DPPH scavenging rate of the sample is calculated as [1 - (A1 - A2)/A3] × 100%.

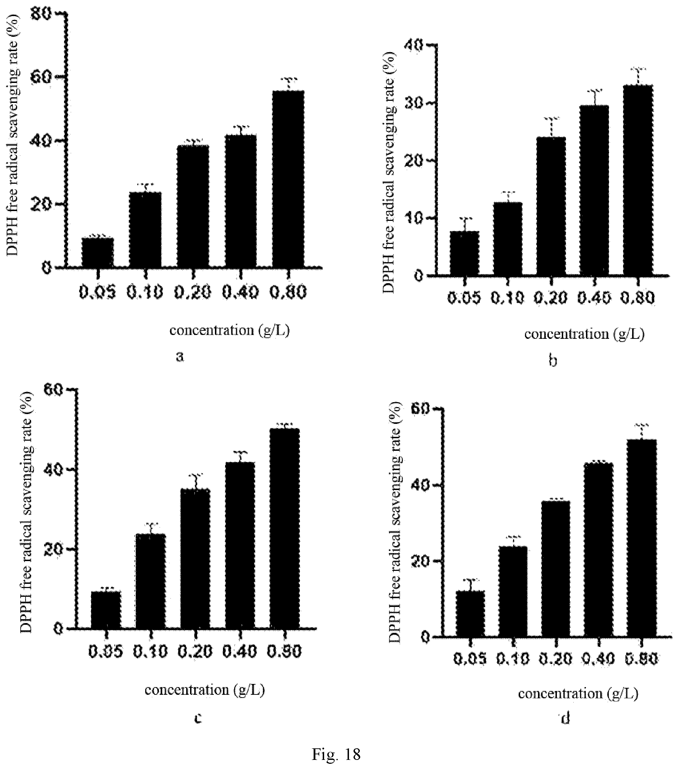

As shown in Figure 18a, the DPPH free radical scavenging rates of camellia oil ceramide at concentrations of 50, 100, 200, 400, and 800 mg/L were 9.43%, 23.91%, 38.53%, 41.93%, and 55.99%, respectively. Using the same method, the antioxidant effect of ceramide 3B (oleic acid ceramide) was tested, and the results (as shown in Figure 18b) showed DPPH scavenging rates of 7.76%, 12.82%, 24.10%, 29.60%, and 33.16% at concentrations of 50, 100, 200, 400, and 800 mg/L, respectively. As shown in Figure 18c, the DPPH scavenging rates of tomato seed oil ceramide at concentrations of 50, 100, 200, 400, and 800 mg/L were 9.43%, 23.91%, 35.10%, 41.93%, and 50.27%, respectively. As shown in Figure 18d, the DPPH scavenging rates of nut oil ceramide at concentrations of 50, 100, 200, 400, and 800 mg/L were 12.09%, 23.91%, 35.82%, 45.82%, and 52.24%, respectively.

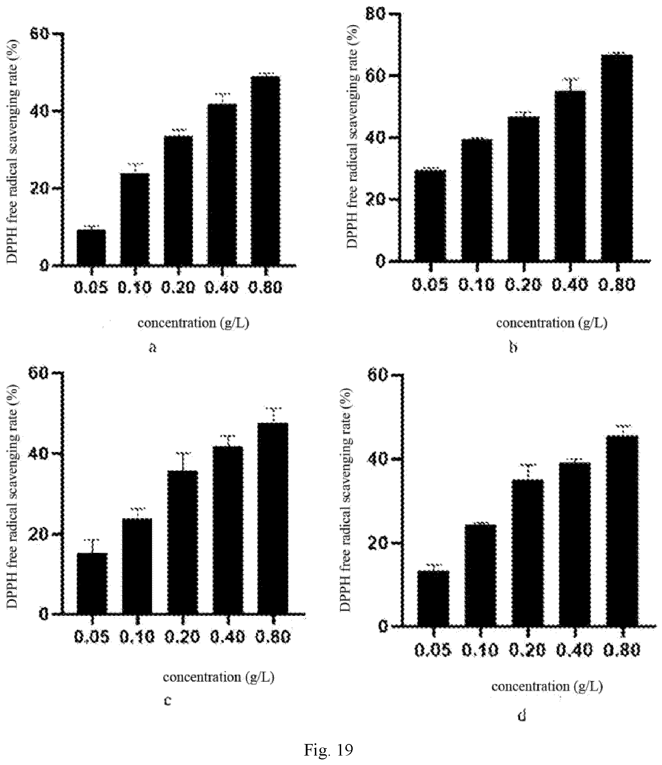

As shown in Figure 19a, the DPPH scavenging rates of cottonseed oil ceramide at concentrations of 50, 100, 200, 400, and 800 mg/L were 9.43%, 23.91%, 33.61%, 41.93%, and 49.19%, respectively. As shown in Figure 19b, the DPPH scavenging rates of olive oil ceramide at concentrations of 50, 100, 200, 400, and 800 mg/L were 29.42%, 39.49%, 46.77%, 55.26%, and 67.01%, respectively. As shown in Figure 19c, the DPPH scavenging rates of argan oil ceramide at concentrations of 50, 100, 200, 400, and 800 mg/L were 15.43%, 23.91%, 35.85%, 41.93%, and 47.56%, respectively. As shown in Figure 19d, the DPPH scavenging rates of cactus seed oil ceramide at concentrations of 50, 100, 200, 400, and 800 mg/L were 13.43%, 24.32%, 35.10%, 39.00%, and 45.57%, respectively.

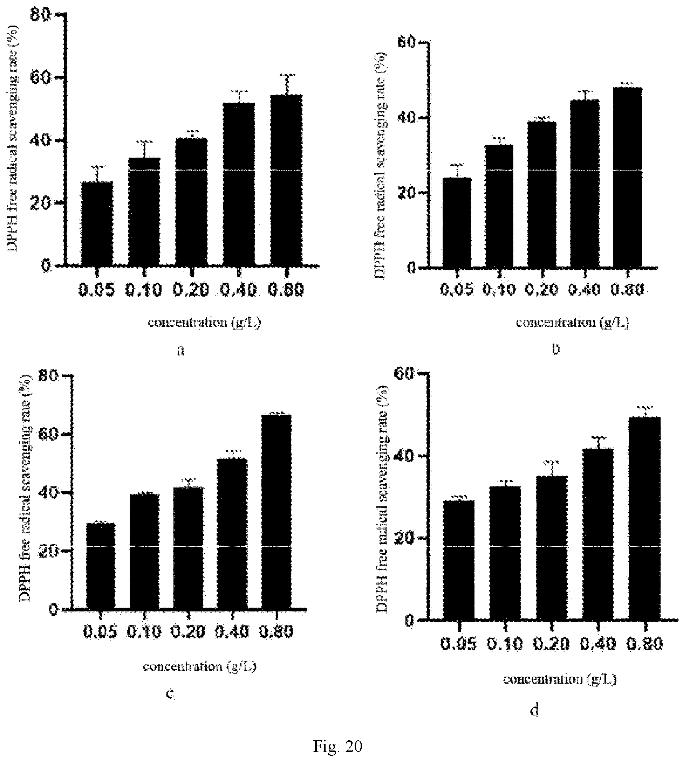

As shown in Figure 20a, the DPPH scavenging rates of sea buckthorn oil ceramide at concentrations of 50, 100, 200, 400, and 800 mg/L were 26.76%, 34.38%, 40.77%, 51.72%, and 54.44%, respectively. As shown in Figure 20b, the DPPH scavenging rates of watermelon seed oil ceramide at concentrations of 50, 100, 200, 400, and 800 mg/L were 24.09%, 32.67%, 39.00%, 44.60%, and 48.13%, respectively. As shown in Figure 20c, the DPPH scavenging rates of evening primrose oil ceramide at concentrations of 50, 100, 200, 400, and 800 mg/L were 29.43%, 39.49%, 41.93%, 51.93%, and 66.83%, respectively. As shown in Figure 20d, the DPPH scavenging rates of coconut oil ceramide at concentrations of 50, 100, 200, 400, and 800 mg/L were 29.09%, 32.72%, 35.10%, 41.93%, and 49.67%, respectively.

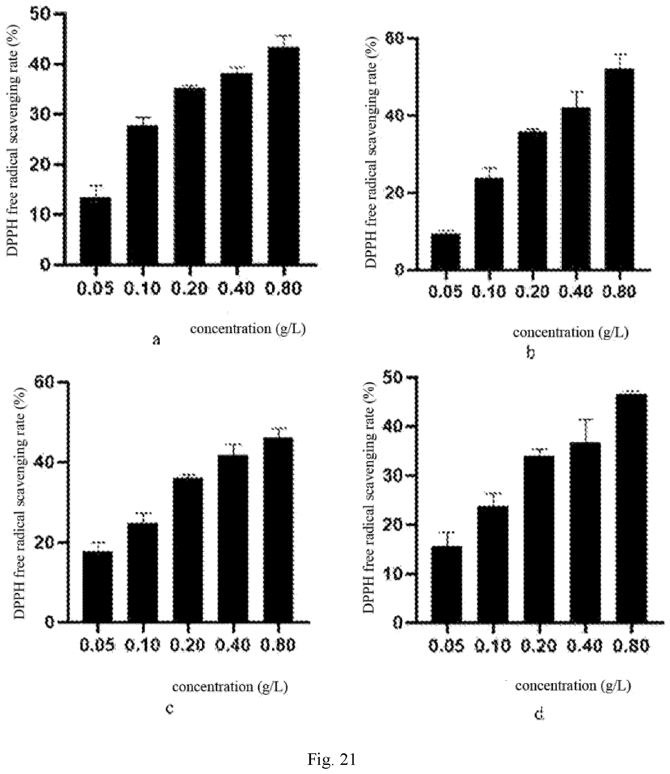

As shown in Figure 21a, the DPPH scavenging rates of soybean oil ceramide at concentrations of 50, 100, 200, 400, and 800 mg/L were 13.43%, 27.91%, 35.24%, 38.26%, and 43.50%, respectively. As shown in Figure 21b, the DPPH scavenging rates of sunflower seed oil ceramide at concentrations of 50, 100, 200, 400, and 800 mg/L were 9.43%, 23.91%, 35.82%, 42.16%, and 52.24%, respectively. As shown in Figure 21c, the DPPH scavenging rates of DHA algal oil ceramide at concentrations of 50, 100, 200, 400, and 800 mg/L were 17.76%, 24.91%, 36.20%, 41.93%, and 46.14%, respectively. As shown in Figure 21d, the DPPH scavenging rates of pumpkin seed oil ceramide at concentrations of 50, 100, 200, 400, and 800 mg/L were 15.76%, 23.91%, 34.09%, 36.77%, and 46.48%, respectively.

Therefore, the plant-derived ceramides of the present invention exhibit higher DPPH scavenging rates than ceramide 3B, demonstrating superior antioxidant efficacy.

### Example 43

### Whitening activity test

B16 cells in the exponential growth phase were digested with 0.25 wt.% trypsin-EDTA and homogenized by pipetting. The cells were seeded into 12-well plates at a density of 3×10⁵ cells per well and cultured overnight at 37°C under 5 wt.% CO₂. The supernatant was discarded, and culture medium containing samples at different concentrations was added. Cells incubated with RPMI-1640 medium without samples served as the blank group, while cells incubated with DMEM medium served as the model group. Each group had three replicate wells and was incubated at 37°C under 5wt.% CO₂ for 24 h. The medium in the wells was discarded, and the cells were washed once or twice with phosphate-buffered saline (PBS). Then, 1 mL of NaOH solution (1 mol/L) containing 10wt.% DMSO was added to lyse the cells, followed by incubation at 80°C or 100°C for 2 h until complete dissolution. The absorbance was measured at 405 nm using a microplate reader. The melanin inhibition rate was calculated as [1 - (OD value of each well / OD value of the model group)] × 100%.

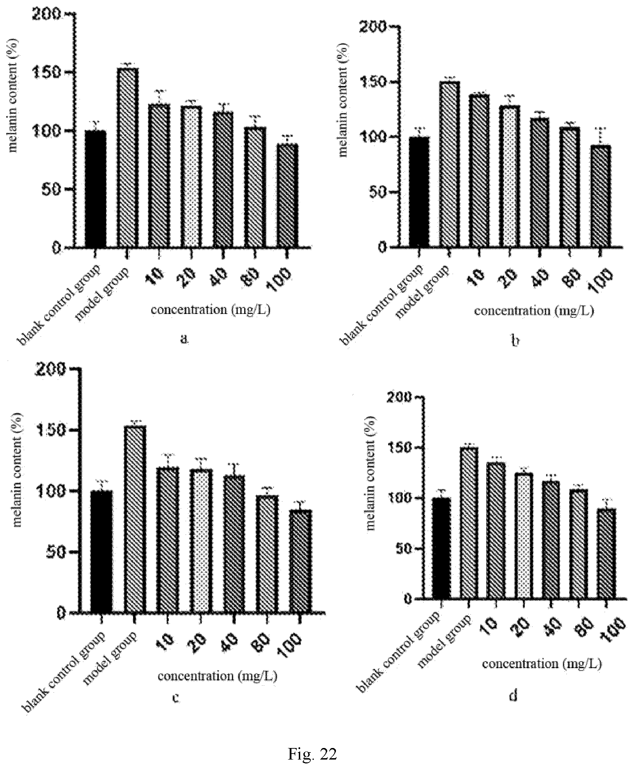

As shown in Figure 22a, with the melanin content of the blank control group was set as 1 and the model group's melanin expression level was 1.54, the melanin inhibition rates of evening primrose oil ceramide at concentrations of 10, 20, 40, 80, and 100 mg/L were 20.08%, 21.12%, 24.33%, 33.03%, and 42.26%, respectively. As shown in Figure 22b, with the blank control group's melanin content was set as 1 and the model group's melanin expression level was 1.51, the melanin inhibition rates of shea butter oil ceramide at concentrations of 10, 20, 40, 80, and 100 mg/L were 8.14%, 14.73%, 22.21%, 27.79%, and 38.33%, respectively. As shown in Figure 22c, with the blank control group's melanin content was set as 1 and the model group's melanin expression level was 1.54, the melanin inhibition rates of *Silybum marianum* oil ceramide at concentrations of 10, 20, 40, 80, and 100 mg/L were 22.24%, 23.28%, 26.49%, 37.36%, and 44.85%, respectively. As shown in Figure 22d, with the blank control group's melanin content was set as 1 and the model group's melanin expression level was 1.51, the melanin inhibition rates of peony seed oil ceramide at concentrations of 10, 20, 40, 80, and 100 mg/L were 9.91%, 16.94%, 22.21%, 27.79%, and 40.54%, respectively.

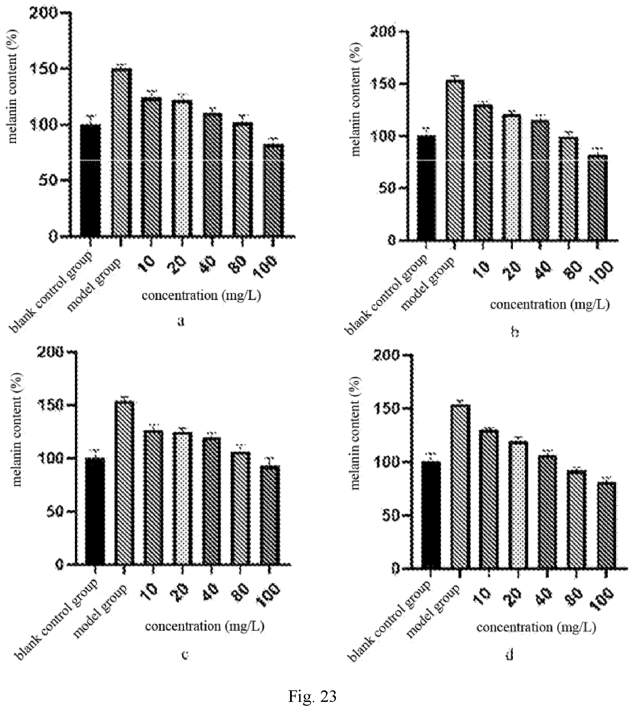

As shown in Figure 23a, with the blank control group's melanin content was set as 1 and the model group's melanin expression level was 1.54, the melanin inhibition rates of tomato seed oil ceramide at concentrations of 10, 20, 40, 80, and 100 mg/L were 13.59%, 21.12%, 28.66%, 38.66%, and 40.53%, respectively. As shown in Figure 23b, with the blank control group's melanin content was set as 1 and the model group's melanin expression level was 1.54, the melanin inhibition rates of *Acer truncatum* oil ceramide at concentrations of 10, 20, 40, 80, and 100 mg/L were 22.24%, 34.10%, 39.48%, 46.02%, and 51.35%, respectively. As shown in Figure 23c, with the blank control group's melanin content was set as 1 and the model group's melanin expression level was 1.51, the melanin inhibition rates of *Plukenetia volubilis* oil ceramide at concentrations of 10, 20, 40, 80, and 100 mg/L were 16.54%, 25.79%, 28.85%, 34.43%, and 42.75%, respectively. As shown in Figure 23d, with the blank control group's melanin content was set as 1 and the model group's melanin expression level was 1.51, the melanin inhibition rates of rosehip oil ceramide at concentrations of 10, 20, 40, 80, and 100 mg/L were 11.68%, 19.15%, 25.53%, 27.79%, and 40.54%, respectively.

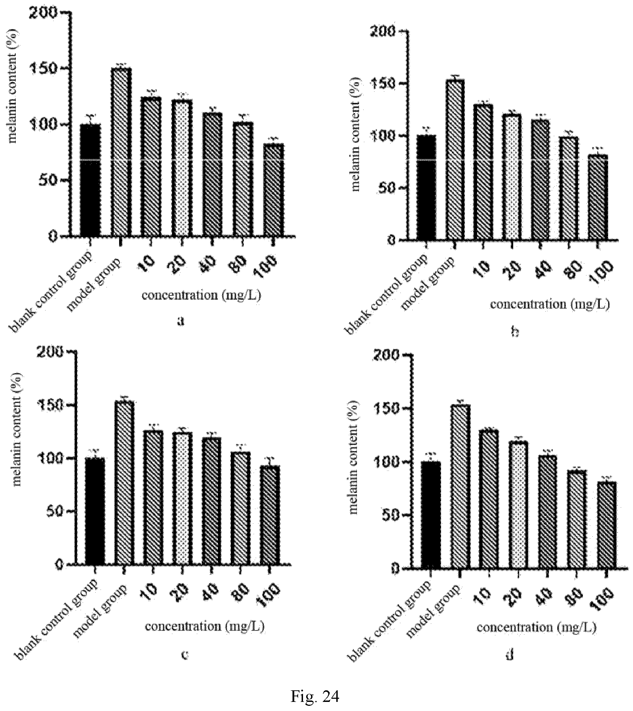

As shown in Figure 24a, with the blank control group's melanin content was set as 1 and the model group's melanin expression level was 1.51, the melanin inhibition rates of grape seed oil ceramide at concentrations of 10, 20, 40, 80, and 100 mg/L were 17.43%, 19.15%, 26.64%, 32.22%, and 44.96%, respectively. As shown in Figure 24b, with the blank control group's melanin content was set as 1 and the model group's melanin expression level was 1.54, the melanin inhibition rates of sea buckthorn oil ceramide at concentrations of 10, 20, 40, 80, and 100 mg/L were 15.10%, 21.55%, 24.98%, 35.20%, and 46.59%, respectively. As shown in Figure 24c, with the blank control group's melanin content was set as 1 and the model group's melanin expression level was 1.54, the melanin inhibition rates of argan oil ceramide at concentrations of 10, 20, 40, 80, and 100 mg/L were 17.91%, 18.95%, 22.16%, 30.87%, and 39.66%, respectively. As shown in Figure 24d, with the blank control group's melanin content was set as 1 and the model group's melanin expression level was 1.54, the melanin inhibition rates of marula oil ceramide at concentrations of 10, 20, 40, 80, and 100 mg/L were 15.53%, 22.42%, 30.82%, 40.39%, and 47.02%, respectively.

Thus, the plant-derived ceramides of the present invention exhibit significant whitening efficacy.

The above descriptions are merely specific embodiments of the present invention. However, the scope of protection of the present invention is not limited to these embodiments. Any modifications or substitutions that can be easily conceived by those skilled in the art within the technical scope disclosed by the invention shall fall within the protection scope of the present invention. Thus, the protection scope of the present invention shall be subject to the claims.

## Claims

1. Plant oil ceramide, prepared by a reaction of a plant oil fatty acid with a sphingoid base compound, wherein the plant oil fatty acid is selected from at least one of olive oil fatty acid, camellia oil fatty acid, sea buckthorn oil fatty acid, grape seed oil fatty acid, nut oil fatty acid, borage oil fatty acid, rosehip oil fatty acid, soybean oil fatty acid, rice bran oil fatty acid, cottonseed oil fatty acid, *Plukenetia volubilis* oil fatty acid, watermelon seed oil fatty acid, coconut oil fatty acid, *Acer truncatum* seed oil fatty acid, walnut oil fatty acid, avocado oil fatty acid, tomato seed oil fatty acid, *Ximenia americana* seed oil fatty acid, *Prinsepia utilis* oil fatty acid, safflower seed oil fatty acid, peony seed oil fatty acid, sunflower seed oil fatty acid, perilla seed oil fatty acid, linseed oil fatty acid, *Silybum marianum* oil fatty acid, shea butter fatty acid, evening primrose oil fatty acid, argan oil fatty acid, hemp seed oil fatty acid, meadowfoam seed oil fatty acid, baobab seed oil fatty acid, cactus seed oil fatty acid, wheat germ oil fatty acid, sesame oil fatty acid, corn oil fatty acid, almond oil fatty acid, *Malania oleifera* oil fatty acid, peanut oil fatty acid, blackcurrant seed oil fatty acid, rapeseed oil fatty acid, hazelnut oil fatty acid, pumpkin seed oil fatty acid, DHA algal oil fatty acid, crabapple oil fatty acid, kiwi seed oil fatty acid, marula oil fatty acid, and European plum seed oil fatty acid.

2. The plant oil ceramide according to claim 1, **characterized in that** the sphingoid base compound is selected from at least one of sphingosine, phytosphingosine, and dihydrosphingosine.

3. The plant oil ceramide according to claim 1, **characterized in that** the plant oil fatty acid is obtained by hydrolysis of a plant oil.

4. The plant oil ceramide according to claim 1, **characterized in that**
the olive oil fatty acid is composed of 20-83 wt.% oleic acid, 7.5-20 wt.% palmitic acid, 3.5-70 wt.% linoleic acid, 0.5-5 wt.% stearic acid, 0.1-1.0 wt.% linolenic acid, 0-3.5 wt.% palmitoleic acid, 0-0.6 wt.% arachidic acid, 0-0.4 wt.% eicosenoic acid, 0-0.3 wt.% heptadecanoic acid, 0-0.3 wt.% heptadecenoic acid, 0-0.2 wt.% behenic acid, 0-0.2 wt.% lignoceric acid, 0-0.05 wt.% myristic acid;
the camellia oil fatty acid is composed of 75-90 wt.% oleic acid, 2-15 wt.% linoleic acid, 1-10 wt.% palmitic acid, 0.1-3 wt.% stearic acid, 0.01-1 wt.% linolenic acid, 0-0.5 wt.% arachidic acid;
the sea buckthorn oil fatty acid is composed of 20-35 wt.% oleic acid, 8-30 wt.% palmitic acid, 4-40 wt.% linoleic acid, 2-30 wt.% linolenic acid, 0.5-35 wt.% palmitoleic acid, 0.5-3 wt.% stearic acid, 0-1 wt.% myristic acid;
the grape seed oil fatty acid is composed of 55-85 wt.% linoleic acid, 8-25 wt.% oleic acid, 1-12 wt.% palmitic acid, 0.5-7 wt.% stearic acid, 0.01-2 wt.% palmitoleic acid, 0.01-1 wt.% linolenic acid, 0-1 wt.% arachidic acid;
the nut oil fatty acid is composed of 55-80 wt.% oleic acid, 12-35 wt.% palmitoleic acid, 2-10 wt.% palmitic acid, 0.4-3 wt.% linoleic acid, 0.1-3 wt.% arachidic acid, 0.2-5 wt.% stearic acid, 0.01-1 wt.% linolenic acid, 0.01-1 wt.% behenic acid, 0-1.5 wt.% myristic acid, 0-3 wt.% arachidonic acid;
the borage oil fatty acid is composed of 20-40 wt.% γ-linolenic acid, 30-45 wt.% linoleic acid, 15-25 wt.% oleic acid, 2-8 wt.% palmitic acid, 1-5 wt.% stearic acid, 0-2 wt.% arachidic acid, 0-2 wt.% arachidonic acid, 0-2 wt.% erucic acid;
the rosehip oil fatty acid is composed of 30-60 wt.% linoleic acid, 20-45 wt.% linolenic acid, 6-25 wt.% oleic acid, 0.1-3 wt.% stearic acid, 0.5-5 wt.% palmitic acid, 0.01-1 wt.% arachidic acid, 0-1 wt.% arachidonic acid;
the soybean oil fatty acid is composed of 50-75 wt.% linoleic acid, 15-30 wt.% oleic acid, 2-12 wt.% palmitic acid, 1-10 wt.% linolenic acid, 1-5 wt.% stearic acid, 0-2 wt.% arachidic acid, 0-2 wt.% behenic acid;
the rice bran oil fatty acid is composed of 40-60 wt.% oleic acid, 20-45 wt.% linoleic acid, 5-20 wt.% palmitic acid, 0.5-5 wt.% stearic acid, 0.1-2 wt.% linolenic acid, 0-2 wt.% arachidic acid;
the cottonseed oil fatty acid is composed of 55-80 wt.% linoleic acid, 10-25 wt.% palmitic acid, 6-18 wt.% oleic acid, 1-3 wt.% stearic acid, 0.1-1 wt.% arachidic acid, 0.02-1 wt.% palmitoleic acid, and 0.01-1 wt.% linolenic acid;
the *Plukenetia volubilis* oil fatty acid is composed of 45-68 wt.% linolenic acid, 20-40 wt.% linoleic acid, 5-12 wt.% oleic acid, 2-5 wt.% palmitic acid, 1-4 wt.% stearic acid;
the watermelon seed oil fatty acid is composed of 60-80 wt.% linoleic acid, 3-15 wt.% palmitic acid, 10-30 wt.% oleic acid, 1-5 wt.% stearic acid;
the coconut oil fatty acid is composed of 20-70 wt.% lauric acid, 10-50 wt.% myristic acid, 10-20 wt.% palmitic acid, 2-10 wt.% stearic acid, 2-10 wt.% oleic acid, 0-3 wt.% decanoic acid, 0-3 wt.% caproic acid;
the *Acer truncatum* seed oil fatty acid is composed of 25-48 wt.% linoleic acid, 20-40 wt.% oleic acid, 5-30 wt.% arachidic acid, 4-15 wt.% arachidonic acid, 0.5-5 wt.% linolenic acid, 0.1-5 wt.% palmitic acid, 0.1-4 wt.% stearic acid;
the walnut oil fatty acid is composed of 40-75 wt.% linoleic acid, 15-50 wt.% oleic acid, 2-10 wt.% palmitic acid, 0-5 wt.% linolenic acid, 0-4 wt.% stearic acid;
the avocado oil fatty acid is composed of 50-80 wt.% oleic acid, 5-25 wt.% palmitic acid, 5-20 wt.% linoleic acid, 6-18 wt.% palmitic acid, 0.1-2 wt.% stearic acid, 0.1-1 wt.% linolenic acid, 0-1 wt.% arachidic acid, 0-0.5 wt.% arachidonic acid;
the tomato seed oil fatty acid is composed of 55-80 wt.% linoleic acid, 10-25 wt.% oleic acid, 3-20 wt.% palmitic acid, 1-8 wt.% stearic acid, 0.2-2 wt.% linolenic acid, 0-1 wt.% arachidic acid;
the *Ximenia americana* seed oil fatty acid is composed of 50-75 wt.% oleic acid, 15-40 wt.% linolenic acid, 0.5-10 wt.% stearic acid, 0-15 wt.% ximenynic acid, 0-15 wt.% nervonic acid, 0-2 wt.% behenic acid, 0-1 wt.% palmitic acid, 0-1 wt.% linoleic acid;
the *Prinsepia utilis* oil fatty acid is composed of 30-50 wt.% linoleic acid, 25-40 wt.% oleic acid, 10-25 wt.% palmitic acid, 3-10 wt.% stearic acid, 0-1 wt.% palmitoleic acid, 0-1 wt.% arachidic acid;
the safflower seed oil fatty acid is composed of 70-90 wt.% linoleic acid, 4-20 wt.% oleic acid, 1-8 wt.% palmitic acid, 0.01-2 wt.% linolenic acid, 0-2 wt.% stearic acid, 0-1 wt.% arachidic acid;
the peony seed oil fatty acid is composed of 35-70 wt.% α-linolenic acid, 15-40 wt.% linoleic acid, 10-30 wt.% oleic acid, 0.1-5 wt.% palmitic acid, 0.1-5 wt.% stearic acid, 0-1 wt.% arachidic acid;
the sunflower seed oil fatty acid is composed of 55-75 wt.% linoleic acid, 20-40 wt.% oleic acid, 0.5-7 wt.% stearic acid, 1-8 wt.% palmitic acid, 0-2 wt.% behenic acid, 0-1 wt.% arachidic acid;
the perilla seed oil fatty acid is composed of 65-90 wt.% α-linolenic acid, 4-30 wt.% linoleic acid, 4-20 wt.% oleic acid, 1-4 wt.% stearic acid, 0-3.5 wt.% palmitic acid, 0-0.6 wt.% arachidonic acid;
the linseed oil fatty acid is composed of 40-70 wt.% linolenic acid, 10-25 wt.% linoleic acid, 10-25 wt.% oleic acid, 1-8 wt.% palmitic acid, 1-8 wt.% stearic acid, 0-1 wt.% arachidic acid;
the *Silybum marianum* oil fatty acid is composed of 50-75 wt.% linoleic acid, 15-45 wt.% oleic acid, 1-10 wt.% palmitic acid, 1-6 wt.% stearic acid, 0.2-4 wt.% arachidic acid, 0.5-4 wt.% behenic acid, 0.01-2.5 wt.% linolenic acid, 0-1.5 wt.% myristic acid;
the shea butter fatty acid is composed of 35-60 wt.% oleic acid, 25-55 wt.% stearic acid, 2-10 wt.% linoleic acid, 0.5-10 wt.% palmitic acid, 0.1-2 wt.% arachidic acid, 0-1 wt.% linolenic acid, 0-1 wt.% behenic acid;
the evening primrose oil fatty acid is composed of 55-88 wt.% linoleic acid, 3-20 wt.% oleic acid, 5-20 wt.% γ-linolenic acid, 0.5-5 wt.% palmitic acid, 0-2 wt.% stearic acid, 0-2 wt.% arachidonic acid, 0-1 wt.% behenic acid;
the argan oil fatty acid is composed of 35-50 wt.% oleic acid, 30-50 wt.% linoleic acid, 5-15 wt.% palmitic acid, 2-8 wt.% stearic acid, 0.1-1 wt.% arachidic acid, 0.01-0.5 wt.% linolenic acid, 0-0.5 wt.% behenic acid, 0-0.5 wt.% arachidonic acid;
the hemp seed oil fatty acid is composed of 50-75 wt.% linoleic acid, 5-30 wt.% linolenic acid, 5-20 wt.% oleic acid, 3-15 wt.% palmitic acid, 1-5 wt.% stearic acid;
the meadowfoam seed oil fatty acid is composed of 50-80 wt.% cis-5-eicosenoic acid, 8-25 wt.% cis-5,13-docosadienoic acid, 5-20 wt.% erucic acid, 1-10 wt.% cis-5-docosenoic acid, 0-1 wt.% oleic acid, 0-1 wt.% linoleic acid;
the baobab seed oil fatty acid is composed of 30-50 wt.% oleic acid, 15-35 wt.% linoleic acid, 13-30 wt.% palmitic acid, 2-8 wt.% stearic acid, 0.5-5 wt.% linolenic acid, 0.2-2 wt.% arachidic acid, 0-1 wt.% palmitoleic acid, 0-0.5 wt.% myristic acid;
the cactus seed oil fatty acid is composed of 50-75 wt.% linolenic acid, 10-30 wt.% oleic acid, 5-15 wt.% palmitoleic acid, 3-10 wt.% palmitic acid, 3-8 wt.% stearic acid, 0.1-1 wt.% arachidic acid;
the wheat germ oil fatty acid is composed of 50-75 wt.% linoleic acid, 15-45 wt.% oleic acid, 2-10 wt.% palmitic acid, 1-8 wt.% linolenic acid, 0.1-1 wt.% arachidonic acid, 0-0.5 wt.% stearic acid, 0-0.5 wt.% arachidic acid;
the sesame oil fatty acid is composed of 45-70 wt.% linoleic acid, 15-35 wt.% oleic acid, 6-20 wt.% palmitic acid, 0-2 wt.% stearic acid, 0-1 wt.% arachidic acid, 0-0.5 wt.% linolenic acid;
the corn oil fatty acid is composed of 50-70 wt.% linoleic acid, 20-35 wt.% oleic acid, 5-18 wt.% palmitic acid, 0.5-3 wt.% stearic acid, 0.01-1 wt.% linolenic acid, 0-1 wt.% arachidic acid;
the almond oil fatty acid is composed of 55-80 wt.% oleic acid, 15-35 wt.% linoleic acid, 0.1-8 wt.% palmitic acid, 0.5-2 wt.% stearic acid, 0.01-1 wt.% linolenic acid;
the *Malania oleifera* oil fatty acid is composed of 40-60 wt.% nervonic acid, 30-50 wt.% oleic acid, 5-12 wt.% arachidic acid, 0-2 wt.% palmitic acid, 0-2 wt.% linoleic acid;
the peanut oil fatty acid is composed of 40-60 wt.% linoleic acid, 30-50 wt.% oleic acid, 4-12 wt.% palmitic acid, 0.5-5 wt.% stearic acid, 0-1 wt.% arachidic acid, 0-1 wt.% behenic acid;
the blackcurrant seed oil fatty acid is composed of 40-60 wt.% linoleic acid, 20-40 wt.% linolenic acid, 10-20 wt.% oleic acid, 1-8 wt.% palmitic acid, 0-2 wt.% stearic acid, 0-2 wt.% arachidic acid;
the rapeseed oil fatty acid is composed of 50-80 wt.% oleic acid, 10-30 wt.% linoleic acid, 2-8 wt.% linolenic acid, 1-5 wt.% stearic acid, 0.5-5 wt.% palmitic acid, 0-1 wt.% arachidic acid, 0-1 wt.% arachidonic acid, 0-1 wt.% erucic acid;
the hazelnut oil fatty acid is composed of 70-90 wt.% oleic acid, 8-25 wt.% linoleic acid, 0.5-5 wt.% palmitic acid, 0.5-4 wt.% stearic acid, 0-0.5 wt.% linolenic acid, 0-0.5 wt.% palmitoleic acid;
the pumpkin seed oil fatty acid is composed of 40-70 wt.% linoleic acid, 20-55 wt.% oleic acid, 3-10 wt.% palmitic acid, 1-5 wt.% stearic acid, 0.1-5 wt.% linolenic acid, 0-1 wt.% arachidic acid, 0-0.5 wt.% palmitoleic acid;
the DHA algal oil fatty acid is composed of 50-75 wt.% DHA, 15-35 wt.% oleic acid, 3-15 wt.% palmitic acid, 0.1-1 wt.% stearic acid, 0.1-1 wt.% EPA, 0.01-0.5 wt.% linolenic acid, 0-0.5 wt.% arachidic acid, 0-0.5 wt.% behenic acid;
the crabapple oil fatty acid is composed of 30-50 wt.% oleic acid, 15-40 wt.% Linoleic acid, 8-20 wt.% stearic acid, 6-20 wt.% palmitic acid, 0.2-1.5 wt.% arachidic acid, 0.05-2 wt.% linolenic acid, 0.05-1 wt.% palmitoleic acid, 0-1 wt.% behenic acid, 0-0.5 wt.% arachidonic acid;
the kiwi seed oil fatty acid is composed of 45-70 wt.% linolenic acid, 8-20 wt.% linoleic acid, 5-20 wt.% oleic acid, 2-10 wt.% palmitic acid, 0.5-5 wt.% stearic acid;
the marula oil fatty acid is composed of 65-85 wt.% oleic acid, 5-15 wt.% palmitic acid, 2-10 wt.% linoleic acid, 2-10 wt.% stearic acid, 0.2-2 wt.% arachidic acid, 0-1 wt.% arachidonic acid, 0-0.5 wt.% linolenic acid; and
the European plum seed oil fatty acid is composed of 60-80 wt.% oleic acid, 10-30 wt.% linoleic acid, 0.5-5 wt.% stearic acid, 0.5-3 wt.% palmitoleic acid, 0-7 wt.% palmitic acid, 0-1 wt.% linolenic acid.

5. Plant oil ceramide, selected from at least two of oleic acid ceramide, linoleic acid ceramide, palmitic acid ceramide, linolenic acid ceramide, stearic acid ceramide, palmitoleic acid ceramide, arachidic acid ceramide, arachidonic acid ceramide, behenic acid ceramide, erucic acid ceramide, myristic acid ceramide, nervonic acid, lauric acid ceramide, eicosenoic acid ceramide, heptadecanoic acid ceramide, heptadecenoic acid ceramide, lignoceric acid ceramide, DHA ceramide, EPA ceramide, decanoic acid ceramide, caproic acid ceramide, ximenynic acid ceramide, cis-5-eicosenoic acid ceramide, cis-5,13-docosadienoic acid ceramide, and cis-5-docosenoic acid ceramide.

6. The plant oil ceramide according to claim 5, selected from at least two of no more than 90 wt.% oleic acid ceramide, no more than 90 wt.% linoleic acid ceramide, no more than 30 wt.% palmitic acid ceramide, no more than 90 wt.% linolenic acid ceramide, no more than 55 wt.% stearic acid ceramide, no more than 35 wt.% palmitoleic acid ceramide, no more than 30 wt.% arachidic acid ceramide, no more than 15 wt.% arachidonic acid ceramide, no more than 4 wt.% behenic acid ceramide, no more than 20 wt.% erucic acid ceramide, no more than 50 wt.% myristic acid ceramide, no more than 60 wt.% nervonic acid ceramide, no more than 70 wt.% lauric acid ceramide, no more than 0.4 wt.% eicosenoic acid ceramide, no more than 0.3 wt.% heptadecanoic acid ceramide, no more than 0.3 wt.% heptadecenoic acid ceramide, no more than 0.2 wt.% lignoceric acid ceramide, no more than 75 wt.% DHA ceramide, no more than 1 wt.% EPA ceramide, no more than 3 wt.% decanoic acid ceramide, no more than 3 wt.% caproic acid ceramide, no more than 15 wt.% ximenynic acid ceramide, no more than 80 wt.% cis-5-eicosenoic acid ceramide, no more than 25 wt.% *cis-5,13-docosadienoic* acid ceramide, and no more than 10 wt.% cis-5-docosenoic acid ceramide.

7. A method for synthesizing the plant oil ceramide according to any one of claims 1-6, comprising the following steps:
under the condition of a condensing agent and an organic base, reacting a plant oil fatty acid with a sphingoid base compound, the condensing agent is DCC or EDCI, and the organic base is DMAP, DIPEA, NMM or Et₃N;
the molar ratio of the plant oil fatty acid, the sphingoid base compound, the condensing agent, and organic base is 1 : (1-1.5) : (1-2) : (0.2-2), and the reaction solvent is at least one of dichloromethane, tetrahydrofuran, ethyl acetate, and acetonitrile.

8. Use of the plant oil ceramide according to any one of claims 1 to 6 in the preparation of cosmetics, pharmaceuticals, dietary products, or health products.

9. The use according to claim 8, **characterized in that** the plant oil ceramide exhibits at least one of the effects of skin barrier repair, tissue healing, anti-aging, anti-inflammatory, anti-photoaging, anti-oxidation, collagen synthesis promotion, maintenance of elastin activity, and skin whitening.

10. A composition, comprising the plant oil ceramide according to any one of claims 1 to 6, wherein the composition has at least one of the effects of skin barrier repair, tissue healing, anti-aging, anti-inflammatory, anti-photoaging, anti-oxidation, collagen synthesis promotion, maintenance of elastin activity, and skin whitening.
